(19) Europäisches Patentamt
European Patent Office
Office européen des brevets

(11) EP 0 695 289 B1

(12) **EUROPÄISCHE PATENTSCHRIFT**

(45) Veröffentlichungstag und Bekanntmachung des Hinweises auf die Patenterteilung: **01.07.1998 Patentblatt 1998/27**

(21) Anmeldenummer: **94916159.0**

(22) Anmeldetag: **15.04.1994**

(51) Int. Cl.[6]: **C07C 259/06**, C07C 259/08, C07C 259/10, C11D 3/39, C11D 3/33, C11D 1/10, C07C 229/16, C07C 229/22, C07C 237/12

(86) Internationale Anmeldenummer: **PCT/EP94/01166**

(87) Internationale Veröffentlichungsnummer: **WO 94/24096 (27.10.1994 Gazette 1994/24)**

(54) **HYDROXAMSÄUREN UND HYDROXAMSÄUREETHER SOWIE IHRE VERWENDUNG ALS KOMPLEXBILDNER**

HYDROXAMIC ACIDS AND HYDROXAMIC ACID ETHERS AND THEIR USE AS COMPLEXING AGENTS

ACIDES HYDROXAMIQUES ET ETHERS D'ACIDES HYDROXAMIQUES ET LEUR UTILISATION COMME AGENTS COMPLEXANTS

(84) Benannte Vertragsstaaten:
**DE FR GB IT NL**

(30) Priorität: **22.04.1993 DE 4313137**

(43) Veröffentlichungstag der Anmeldung: **07.02.1996 Patentblatt 1996/06**

(73) Patentinhaber:
**BASF AKTIENGESELLSCHAFT
67056 Ludwigshafen (DE)**

(72) Erfinder:
- **GREINDL, Thomas
D-94127 Neuburg (DE)**
- **KUD, Alexander
D-55234 Eppelsheim (DE)**
- **SCHWENDEMANN, Volker
D-67434 Neustadt (DE)**
- **KNEIP, Michael
D-67227 Frankenthal (DE)**
- **KAPPES, Elisabeth
D-68163 Mannheim (DE)**
- **BAUR, Richard
D-67112 Mutterstadt (DE)**
- **SCHNEIDER, Juergen
D-67251 Freinsheim (DE)**
- **POTTHOFF-KARL, Birgit
D-67061 Ludwigshafen (DE)**
- **OFTRING, Alfred
D-67098 Bad Duerkheim (DE)**

(56) Entgegenhaltungen:
**EP-A- 0 384 911** **EP-A- 0 384 912**
**EP-A- 0 458 131** **WO-A-91/08191**
**US-A- 3 278 649** **US-A- 3 707 502**
**US-A- 4 263 322** **US-A- 5 093 040**
**US-A- 5 316 898**

- **POLYHEDRON Bd. 9, Nr. 2-3 , 1990 , OXFORD GB Seite 151-161 D. J. CLEVETTE, C. ORVIG 'Comparison of ligands of differing denticity and basicity for the in vivo chelation of aluminium and gallium.'**
- **JOURNAL OF BIOLOGICAL CHEMISTRY Bd. 260, Nr. 26 , 1985 , BALTIMORE, MD US Seiten 13916 - 13920 G. MUELLER ET AL. 'Coordination chemistry of microbial iron transport compounds. 31. The mechanism and specificity of iron transport in Rhodotorula pilimanae probed by synthetic analogs of rhodotorulic acid'**
- **INORGANIC CHEMISTRY Bd. 25, Nr. 21 , 1986 , EASTON US Seiten 3792 - 3796 D. A. BROWN ET AL 'Design of metal chelates with biological activity. 5. Complexation behaviour of dihydroxamic acids with metal ions'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

- CHEMICAL ABSTRACTS, vol. 51, no. 3, 10. Februar 1957, Columbus, Ohio, US; abstract no. 2169b, M. A. EPSTEIN ET AL. & EXPTL. BIOL. MED. Bd. 92 , 1956 Seiten 660 - 662
- CHEMICAL ABSTRACTS, vol. 85, no. 9, 30. August 1976, Columbus, Ohio, US; abstract no. 62509, L. G. SHKOLYAR ET AL. & ZH. FIZ. KHIM. Bd. 50, Nr. 4 , 1976 Seite 1051
- CHEMICAL ABSTRACTS, vol. 98, no. 22, 30. Mai 1983, Columbus, Ohio, US; abstract no. 190619c, I. BENEDIKOVIC ET AL. & ACTA FAC. PHARM. UNIV. COMENIANAE Bd. 36 , 1981 Seiten 25 - 67
- CHEMICAL ABSTRACTS, vol. 111, no. 20, 13. November 1989, Columbus, Ohio, US; abstract no. 181983b, M. SIVAK ET AL. & TRANSITION MET. CHEM. Bd. 14, Nr. 4 , 1989 , LONDON Seiten 273 - 6
- CHEMICAL ABSTRACTS, vol. 69, no. 17, 21. Oktober 1968, Columbus, Ohio, US; abstract no. 67681r, B. E. LEACH ET AL. & JOURNAL OF THE AMERICAN CHEMICAL SOCIETY Bd. 90, Nr. 10 , 1968 , WASHINGTON, DC US Seiten 2504 - 2508

## Beschreibung

Die vorliegende Erfindung betrifft die Verwendung von Hydroxamsäuren und Hydroxamsäureether der allgemeinen Formel III und IV

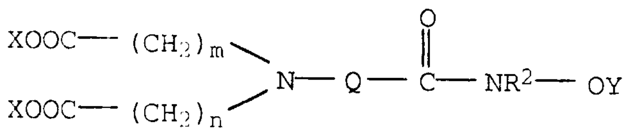

(III)

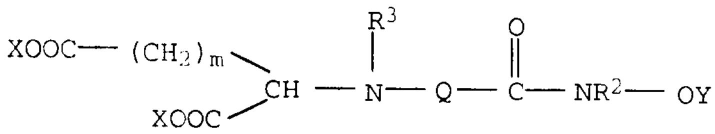

(IV)

in denen

| | |
|---|---|
| $R^2$ | Wasserstoff oder $C_1$- bis $C_{18}$-Alkyl bezeichnet, |
| $R^3$ | Wasserstoff oder eine Gruppierung der Formel —Q—CO—$NR^2$—OY bedeutet, |
| Q | für eine $C_1$- bis $C_8$-Alkylengruppe, insbesondere $C_1$- bis $C_4$-Alkylengruppe, steht, welche zusätzlich bis zu 8, insbesondere bis zu 4 Hydroxylgruppen tragen kann, |
| m | und n jeweils für die Zahl 1 oder 2 stehen, |
| X | Wasserstoff, Alkalimetall, Ammonium oder substituiertes Ammonium bezeichnet und |
| Y | Wasserstoff, Alkalimetall, Ammonium, substituiertes Ammonium oder $C_1$- bis $C_{18}$-Alkyl bedeutet, |

als Gerüststoff und Bleichstabilisatoren in Wasch- und Reinigungsmitteln.

Weiterhin betrifft die Erfindung ein Verfahren zur Herstellung eines Teils der Verbindungen III, die Verbindungen III und IV enthaltende Wasch- und Reinigungsmittel selbst sowie Mischungen der Verbindungen III und IV mit Polycarboxylaten.

Komplexbildner für Erdalkalimetallionen und Schwermetallionen, beispielsweise Calcium, Magnesium, Mangan oder Kupfer, werden für die verschiedensten technischen Gebiete benötigt. Als Einsatzgebiete und Verwendungszwecke kommen beispielsweise in Betracht: Wasch- und Reinigungsmittel, technische Anwendungen für Industriereiniger, in der Galvanotechnik, in der Wasserbehandlung und bei Polymerisationen, die photographische Industrie, die Textilindustrie und die Papierindustrie sowie verschiedene Anwendungen in Pharmazeutika, in der Kosmetik, bei Nahrungsmitteln und bei der Pflanzenernährung.

Dem Fachmann geläufige und anerkannte Komplexbildner sind beispielsweise Nitrilotriessigsäure (NTA), Ethylendiamintetraessigsäure (EDTA), Ethylendiamintetramethylenphosphonsäure (EDTMP), Propylendiamintetraessigsäure (PDTA), Hydroxypropylendiamintetraessigsäure (HPDTA), Isoserindiessigsäure (ISDA), β-Alanindiessigsaure (β-ADA), Hydroxyethandiphosphonsäure, Diethylentriamintetraessigsaure, Diethylentriamintetramethylenphosphonsäure, Hydroxyethyleniminodiessigsaure, Hydroxyethylethylendiamintriessigsäure, Diethylentriaminpentaessigsäure sowie weiterhin Diethanolglycin, Ethanolglycin, Citronensäure, Glucoheptonsäure oder Weinsäure.

Die Wirkung der bekannten und teilweise in großem Maßstab eingesetzten Verbindungen ist im Einzelfall nicht immer optimal. Beispielsweise ist die Wirkung von NTA als Gerüststoff in Wasch- und Reinigungsmitteln zur Verbesserung der Weißwaschwirkung und zur Verhinderung von Ablagerungen, die Inkrustationen und Vergrauung auf dem Gewebe verursachen, noch verbesserungsbedürftig. Die Wirkung von NTA als Bleichmittelstabilisator ist auch nur schwach ausgeprägt. Auch EDTA zeigt trotz seines guten Komplexiervermögens gegenüber Schwermetallen auch eine nur maßige Wirkung als Bleichmittelstabilisator in Wasch- und Reinigungsmitteln und seine Wirkung als Gerüststoff zur Verbesserung der Weißwaschwirkung ist ebenfalls verbesserungsbedürftig.

In manchen Fällen läßt auch die biologische Abbaubarkeit zu wünschen übrig. So erweist sich EDTA in den üblichen Tests als unzureichend biologisch abbaubar, ebenso PDTA oder HPDTA sowie entsprechende Aminomethylenphosphonate, die zudem wegen ihres Phosphorgehaltes oft unerwünscht sind.

In der EP-A 458 131 (1) wird auf S. 11 als Beispiel 4 die Verbindung N,N-Bis(carboxymethyl)-2-aminoacethydroxamsäure als Zusatz zu photographischen Entwicklerbädern empfohlen.

Aus der nachveröffentlichten Schrift EP-A 563 571 (2) (benannte Vertragsstaaten: DE, FR, GB und NL) sind die Hydroxamsäuren der Formel

$HOOC-CH_2-NH-CH_2-CO-NH-OH$,

$HOOC-CH_2-N(CH_3)-CH_2-CO-NH-OH$,

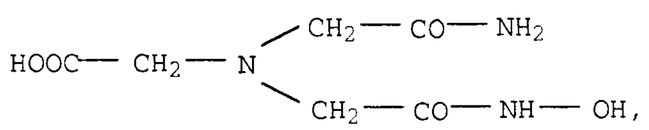
,

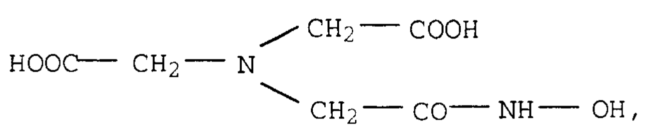
,

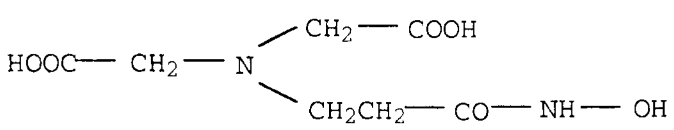
und

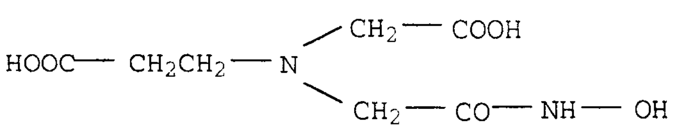

als Bleichmittel für die Silberhalogenid-Farbphotographie bekannt. Diese Verbindungen werden als Eisen-Komplex eingesetzt.

In der US-A 4 822 886 (3) werden cyclische N-Hydroxyimid-Strukturen, welche aus Citronensäureestern und Hydroxylamin in Gegenwart starker Basen hergestellt werden, als Waschmitteladditive beschrieben.

Aus der EP-A 384 912 (4) sind Hydroxamate wie Alkylhydroxamate und Succinomonohydroxamate als Bleichstabilisatoren für Peroxoverbindungen enthaltende Bleichsysteme bekannt.

Aufgabe der vorliegenden Erfindung war es, neue Komplexbildner für Wasch- und Reinigungsmittel zur Verfügung zu stellen, die neben verbesserten Gerüststoff- und Bleichmittelstabilisatoreigenschaften ökologisch unbedenklich sind, moglichst keinen Phosphor enthalten und biologisch gut abbaubar bzw. eliminierbar sind Für diese neuen Komplexbildner sollte auch ein technisch realisierbares Herstellverfahren entwickelt werden.

Demgemäß wurden die eingangs definierten Hydroxamsäuren und Hydroxamsäureether III und IV gefunden.

Die erfindungsgemäßen Verbindungen III bzw. IV haben als gemeinsames Strukturmerkmal, daß sie mindestens drei sauerstoffunktionelle polare Gruppen aufweisen, die als komplexierende Zentren für Erdalkali- und Schwermetallionen wirken. Eine dieser Gruppen ist immer eine Hydroxamsäurefunktion, die in veretherter Form vorliegen kann; die anderen Gruppen sind Carboxylgruppen, die in der freien Säureform oder als Salz vorliegen, eine weitere Hydroxamsäure(ether)funktion oder eine Hydroxylgruppe.

Die Hydroxamsäurefunktion in den Verbindungen III und IV kann auch in einer oxidierten Form (entsprechend der Formel —CO—N=O) vorliegen.

Der Rest $R^2$ bezeichnet neben Wasserstoff lineare oder verzweigte $C_1$- bis $C_{18}$-Alkylgruppen wie Methyl, Ethyl, n-Propyl, iso-Propyl, n-Butyl, iso-Butyl, sek.-Butyl, tert.-Butyl, n-Amyl, iso-Amyl, tert.-Amyl, n-Hexyl, n-Heptyl, n-Octyl, 2-Ethylhexyl, n-Nonyl, iso-Nonyl, n-Decyl, n-Undecyl, n-Dodecyl, n-Tridecyl, iso-Tridecyl, n-Tetradecyl, n-Hexadecyl oder n-Octadecyl. $R^2$ bezeichnet auch gesättigte oder ungesättigte langkettige Fettsäurereste wie Palmityl, Stearyl, Oleyl,

Linolyl oder Linolenyl. Bevorzugt werden für $R^2$ $C_1$- bis $C_8$-Alkylreste, insbesondere $C_1$- bis $C_4$-Alkylreste, vor allem Methyl und Ethyl.

Für die Variablen X und Y kommen neben Wasserstoff Alkalimetall, z.B. Lithium, Natrium oder Kalium, Ammonium oder substituiertes Ammonium wie Trialkylammonium mit jeweils 1 bis 4 C-Atomen im Alkylrest, z.B. Trimethyl- oder Triethylammonium, oder Trialkanolammonium mit jeweils 2 oder 3 C-Atomen im Alkanolrest, z.B. Triethanol-, Tri-n-propanol- oder Triisopropanolammonium, in Betracht. Bedeutet Y $C_1$- bis $C_{18}$-Alkyl, gilt über die diesbezüglichen Bedeutungen das für $R^2$ Gesagte in analoger Weise.

Typische Beispiele für Hydroxamsäuren bzw. Hydroxamsäureether III und IV sind:

| | |
|---|---|
| 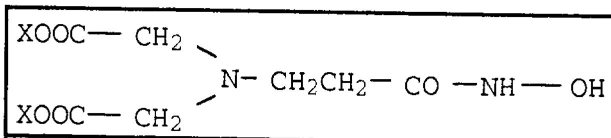 | (N,N-Bis(carboxymethyl)-3-aminopropiohydroxamsäure) |
| 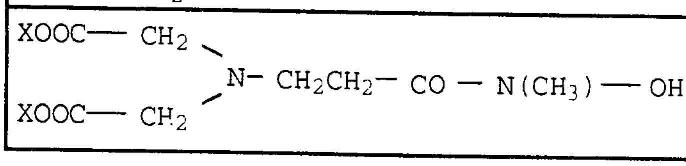 | (N,N-Bis(carboxymethyl)-3-amino-propio-N'-methyl-hydroxamsäure) |

| | |
|---|---|
| 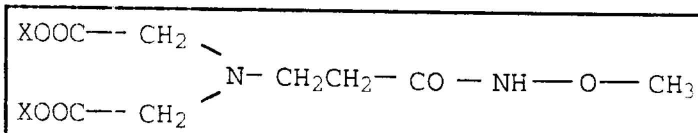 | (N,N-Bis(carboxymethyl)-3-aminopropiohydroxamsäure-O-methylether) |
| 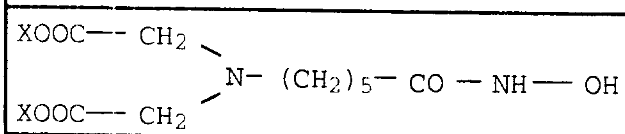 | |
| 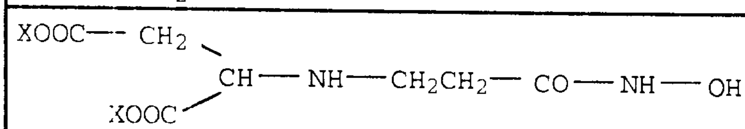 | (Asparginsäure-N-propiohydroxamsäure) |
| 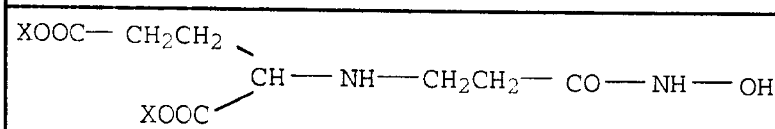 | (Glutaminsäure-N-propiohydroxamsäure) |
| 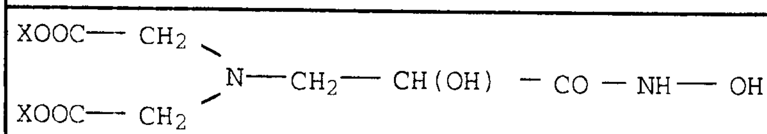 | (N,N-Bis(carboxymethyl)-3-amino-2-hydroxy-propio-hydroxamsäure) |
| 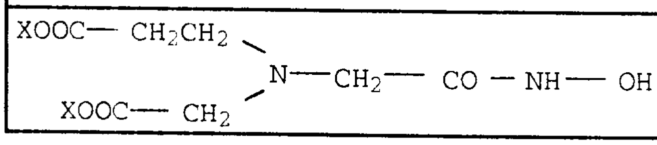 | |

Ganz besonders bevorzugt werden N,N-Bis(carboxymethyl)-3-aminopropiohydroxamsäuren und deren Ether der allgemeinen Formel IIIa

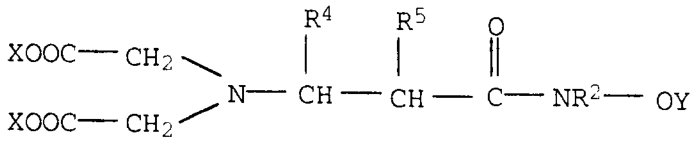

(IIIa)

in der

| | |
|---|---|
| $R^4$ | für Wasserstoff, Methyl oder Ethyl steht, |
| $R^5$ | für Wasserstoff, Methyl, Ethyl oder Hydroxyl steht und |
| $R^2$, X und Y | die oben genannten Bedeutungen haben. |

Die Verbindungen IIIa liegen vorzugsweise als freie Säuren (X = Y = H), als Mononatrium- oder Monokaliumsalz an einer Carboxylgruppe (ein X = Na oder K, das andere X = Y = H), als Dinatrium- oder Dikaliumsalze an beiden Carboxylgruppen (X = Na oder K, Y = H) oder als Trinatrium- oder Trikaliumsalze (X = Y = Na oder K) vor.

Die erfindungsgemäß verwendeten Hydroxamsäuren bzw. deren Ether III, IV und IIIa lassen sich zweckmäßigerweise dadurch herstellen, daß man die zugrundeliegenden Carbonsäure-$C_1$- bis $C_4$-alkylester oder Carbonsäureamide, welche am Amidstickstoff ein oder zwei $C_1$- bis $C_4$-Alkylgruppen tragen können, mit einem Hydroxylamin bzw. einem Hydroxylamin-$C_1$- bis $C_{18}$-alkylether umsetzt und das Produkt gewünschtenfalls durch Behandlung mit den X bzw. Y zugrundeliegenden Basen in die Salzform überführt.

Die Verbindungen IIIa lassen sich in vorteilhafter Weise dadurch herstellen, daß man Iminodiessigsäure der Formel VI

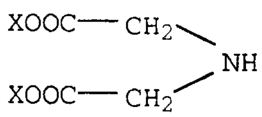

(VI)

mit einem $\alpha,\beta$-ungesättigten Carbonsäureester der allgemeinen Formel VII

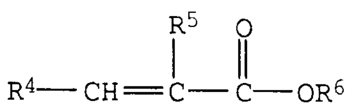

(VII)

oder einem $\alpha,\beta$-ungesättigten Carbonsäureamid der allgemeinen Formel VIII

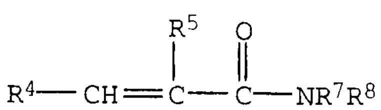

(VIII)

zum N,N-Bis(carboxymethyl)-3-aminopropionsäureester der allgemeinen Formel IX

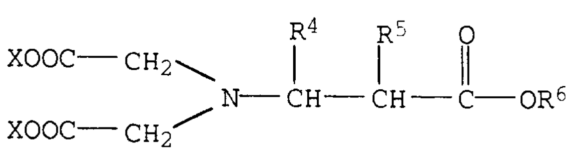

(IX)

bzw. zum N,N-Bis(carboxymethyl)-3-aminopropionsäureamid der allgemeinen Formel X

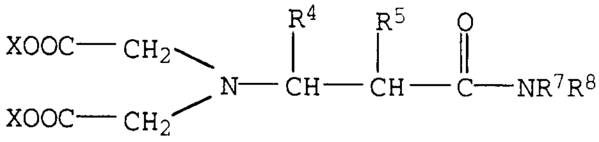

(X)

umsetzt und anschließend aus (IX) bzw. (X) durch Umsetzung mit einem Hydroxylamin oder einem Hydroxylamin-$C_1$- bis $C_{18}$-alkylether das Endprodukt IIIa herstellt, welches gewünschtenfalls durch Behandlung mit den X bzw. Y zugrundeliegenden Basen in die Salzform übergeführt werden kann, wobei die Variablen $R^4$, $R^5$, X und Y die oben genannten Bedeutungen haben sowie $R^6$ $C_1$- bis $C_4$-Alkyl bedeutet und $R^7$ und $R^8$ Wasserstoff oder $C_1$- bis $C_4$-Alkyl bezeichnen.

Als $\alpha,\beta$-ungesättigten Carbonsäureester VII eignen sich beispielsweise der n-Propyl-, iso-Propyl-, n-Butyl-, iso-Butyl-, sec.-Butyl-, tert.-Butyl-, insbesondere jedoch der Ethyl- und ganz besonders bevorzugt der Methylester der Acrylsäure, Methacrylsäure, 2-Ethylacrylsäure, Crotonsäure, Isocrotonsäure, 2-Pentensäure, 2-Methylcrotonsäure oder 2-Ethylcrotonsäure.

Als $\alpha,\beta$-ungesättigten Carbonsäureamide VIII eignen sich beispielsweise die N-unsubstituierten oder die N-mono- oder N,N-dimethyl-substituierten Amide der genannten $\alpha,\beta$-ungesättigten Säuren.

Wegen der leichteren Handhabbarkeit wird Hydroxylamin vorzugsweise in Form eines seiner Salze wie Hydroxylamin-Hydrochlorid oder Hydroxylammoniumsulfat oder als Lösung in einem organischen Lösungsmittel, z.B. Ethanol oder Methanol, eingesetzt.

Als X bzw. Y zugrundeliegende Basen kommen beispielsweise Natriumhydroxid, Natriumcarbonat, Kaliumhydroxid, Kaliumcarbonat, Lithiumhydroxid, Ammoniak, Tri-$C_1$- bis $C_4$-alkylamine wie Trimethyl- oder Triethylamin oder Tri-$C_2$- bis $C_3$-alkanolamine wie Triethanol-, Tri-n-propanol- oder Triisopropanolamin in Betracht.

Die beiden Reaktionsschritte Addition von VI an VII bzw. VIII zu IX bzw. X und Umsetzung von IX bzw. X mit Hydroxylamin können hintereinander mit oder ohne Isolierung des Zwischenproduktes IX bzw. X durchgeführt werden. Da der erste Schritt zweckmäßigerweise in wäßrigem Medium vorgenommen wird, kann bei Isolierung von IX bzw. X (Zweistufenvariante) die weitere Umsetzung in Abwesenheit von Wasser in einem organischen Lösungsmittel, z.B. einem Alkohol wie Methanol oder Ethanol, durchgeführt werden, was in vielen Fällen zu einem etwas reinerem Produkt IIIa führt. Ohne Isolierung von IX bzw. X (Einstufenvariante) kann die Umsetzung von VI zu IIIa dagegen in demselben wäßrigen Medium bis zum Schluß erfolgen, was aus ökonomischer und auch aus ökologischer Sicht (Verzicht auf organische Lösungsmittel) vorteilhafter ist.

Als wäßriges Medium eignet sich insbesondere Wasser, daneben aber auch Mischungen aus Wasser und wassermischbaren organischen Lösungsmitteln wie Methanol, Ethanol, n-Propanol, iso-Propanol, tert.-Butanol, Dioxan oder Tetrahydrofuran.

Die Umsetzung erfolgt zweckmäßigerweise für beide Reaktionsschritte bei Temperaturen von 0 bis 80°C, vorzugsweise von 10 bis 60°C, und bei Normaldruck; es kann aber auch bei erhöhtem Druck gearbeitet werden.

Man arbeitet in der Regel bei pH-Werten von 4 bis 10, vorzugsweise von 5 bis 9, insbesondere von 6 bis 8. Die Fahrweise in der Nähe des Neutralpunktes ist insbesondere dann angebracht, wenn man von dem Monosalz von VI ausgeht. Zur Überführung in die entsprechenden Salzformen oder die Formen der freien Säure des Zwischenproduktes IX bzw. X oder des Endproduktes IIIa muß mit geeigneten Basen oder Säuren behandelt werden.

Man setzt die miteinander umzusetzenden Verbindungen vorzugsweise in stöchiometrischem oder annähernd stöchiometrischem Verhältnis ein, d.h. im molaren Verhältnis VI : VII bzw. VIII = 1 : 1, wobei Abweichungen in den Mengen einzelner Komponenten von bis zu 5 % noch tolerabel sind. Das bevorzugte molare Verhältnis von IX bzw. X zu Hydroxylamin ist ebenfalls 1 : 1, jedoch kann Hydroxylamin auch in größerem Überschuß eingesetzt werden.

Die Verbindungen IIIa können ohne Schwierigkeiten in ausreichend reiner Form isoliert werden. Für die freie Säure und die Salze bieten sich insbesondere Sprüh- oder Gefriertrocknung, Kristallisation oder Fällung an. Es kann vorteilhaft sein, die angefallene Lösung direkt einer technischen Verwendung zuzuführen.

Da die Verbindungen III, IV und IIIa teilweise neue Substanzen darstellen, sind auch Gegenstand der vorliegenden Erfindung Hydroxamsäuren und Hydroxamsäureether der allgemeinen Formel III und IV

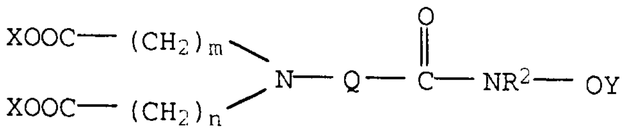

(III)

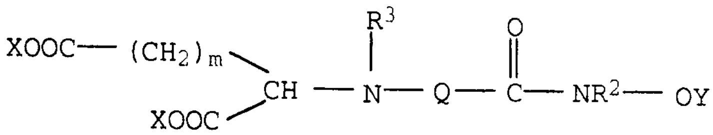

(IV)

in denen

$R^2$ Wasserstoff oder $C_1$- bis $C_{18}$-Alkyl bezeichnet,

$R^3$ Wasserstoff oder eine Gruppierung der Formel —Q—CO—$NR^2$—OY bedeutet,

Q für eine $C_1$- bis $C_8$-Alkylengruppe steht, welche zusätzlich bis zu 8 Hydroxylgruppen tragen kann,

m und n jeweils für die Zahl 1 oder 2 stehen,

X Wasserstoff, Alkalimetall, Ammonium oder substituiertes Ammonium bezeichnet und

Y Wasserstoff, Alkalimetall, Ammonium, substituiertes Ammonium oder $C_1$- bis $C_{18}$-Alkyl bedeutet,

mit Ausnahme von N,N-Bis(carboxymethyl)-2-aminoacethydroxamsäure,
N-Carboxymethyl-N-(β-carboxyethyl)-2-aminoacethydroxamsäure und
N,N-Bis(carboxymethyl)-3-aminopropiohydroxamsäure, sowie
N,N-Bis(carboxymethyl)-3-aminopropiohydroxamsäuren und deren Ether der allgemeinen Formel IIIa

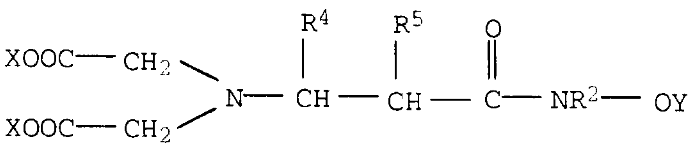

(IIIa)

in der

$R^4$ für Wasserstoff, Methyl oder Ethyl steht,

$R^5$ für Wasserstoff, Methyl, Ethyl oder Hydroxyl steht,

$R^2$ Wasserstoff oder $C_1$- bis $C_{18}$-Alkyl bezeichnet,

X Wasserstoff, Alkalimetall, Ammonium oder substituiertes Ammonium bezeichnet und

Y Wasserstoff, Alkalimetall, Ammonium, substituiertes Ammonium oder $C_1$- bis $C_{18}$-Alkyl bedeutet,

mit Ausnahme von N,N-Bis(carboxymethyl)-3-aminopropiohydroxamsäure.

Die erfindungsgemäß verwendeten Hydroxamsäuren und Hydroxamsäureether III und IV sind in hervorragender Weise geeignet, Erdalkali- und Schwermetallionen, insbesondere Calcium-, Magnesium-, Kupfer-, Eisen- und Manganionen, zu komplexieren. Aufgrund dieser Fähigkeit weisen sie eine Vielzahl von technischen Anwendungsmöglichkeiten auf. Da es sich um biologisch gut abbaubare bzw. eliminierbare Verbindungen handelt, können sie in großen Mengen überall dort eingesetzt werden, wo die Abwässer geklärt werden müssen und auch phosphorhaltige Verbindungen vermieden werden sollen.

In Wasch- und Reinigungsmitteln können die erfindungsgemäß verwendeten Komplexbildner eingesetzt werden, um den Gehalt an freien Schwermetallionen in den Waschmitteln selbst und in den Waschlösungen zu kontrollieren. Die Einsatzmenge als Komplexbildner beträgt zweckmäßigerweise 0,1 bis 2 %, bezogen auf das Gesamtgewicht der Waschmittelbestandteile.

Ihre vorteilhafte Wirkung liegt in einer Bleichmittelstabilisierung, beispielsweise für Natriumperborat, in Waschmitteln und bei der Bleiche von Textilien, Zellstoff oder Papierrohstoff. Spuren von Schwermetallen, wie Eisen, Kupfer und Mangan, kommen im Waschpulver selbst, im Wasser und im Textilgut vor und katalysieren die Zersetzung des Natriumperborates. Die erfindungsgemäß verwendeten Komplexbildner binden diese Metallionen und verhindern die unerwünschte Zersetzung des Bleichsystems während der Lagerung und in der Waschflotte. Dadurch erhöht sich die Effizienz des Bleichsystems und Faserschädigungen werden zurückgedrängt.

Zusätzlich werden Enzyme, optische Aufheller und Duftstoffe vor schwermetall-katalysierter oxidativer Zersetzung geschützt.

In flüssigen Waschmittelformulierungen können die beschriebenen Komplexbildner als sogenannte Konservierungsmittel, zweckmäßig in einer Menge von 0,05 bis 1 Gew.-%, bezogen auf das Gesamtgewicht der Waschmittelformulierung, eingesetzt werden.

In Seifen verhindern die beschriebenen Komplexbildner beispielsweise metall-katalysierte oxidative Zersetzungen.

Weiterhin dienen sie in hervorragender Weise in Wasch- und Reinigungsmitteln als Gerüststoff (Builder), um Ausfällungen und Inkrustationen auf dem Gewebe zu verhindern.

Sie können in vorteilhafter Weise überall dort eingesetzt werden, wo bei technischen Verfahren Ausfällungen von Calcium-, Magnesium- und Schwermetallsalzen stören und verhindert werden sollen. Beispielsweise zur Verhinderung von Ablagerungen und Verkrustungen in Kesseln, Rohrleitungen, an Sprühdüsen oder allgemein an glatten Oberflächen.

Sie können zur Stabilisierung von Phosphaten in alkalischen Entfettungsbädern und Verhinderung der Ausfällung von Kalkseifen dienen und verhindern dadurch das "Anlaufen" von Nichteisenoberflächen und verlängern die Standzeiten von alkalischen Reinigerbädern.

Sie können als Komplexbildner in alkalischen Entrostungs- und Entzunderungsbädern verwendet werden sowie in galvanischen Bädern anstelle von Cyaniden, um Verunreinigungen zu maskieren.

Die Kühlwasserbehandlung mit den beschriebenen Komplexbildern verhindert Ablagerungen bzw. löst bereits vorhandene wieder auf. Vorteil ist die Anwendung in alkalischem Medium und damit die Beseitigung von Korrosionsproblemen.

Bei der Polymerisation von Kautschuk können sie zur Herstellung der dabei verwendeten Redoxkatalysatoren verwendet werden. Sie verhindern zusätzlich das Ausfallen von Eisenhydroxid im alkalischen Polymerisationsmilieu.

In der photographischen Industrie können die beschriebenen Komplexbildner in Bleich-, Fixier- und Bleichfixierbädern, die mit hartem Wasser angesetzt werden, verwendet werden, um die Ausfällung schwerlöslicher Calcium- und Magnesiumsalze zu verhindern. Die Ausfällungen führen zu Grauschleiern auf Filmen und Bildern sowie Ablagerungen in den Tanks, die somit vorteilhaft vermieden werden können. Als Eisen-III-Komplexbildnerlösungen können sie vorteilhaft in Bleichfixierbädern eingesetzt werden, wo sie die aus ökologischen Gründen bedenklichen Hexacyanoferratlösungen ersetzen können.

In der Textilindustrie können sie zur Entfernung von Schwermetallspuren während des Herstellungs- bzw. Färbeprozesses von natürlichen und synthetischen Fasern dienen. Dadurch werden viele Störungen verhindert: Schmutzflecken und Streifen auf dem Textilgut, Verlust des Glanzes, schlechte Benetzbarkeit, unegale Färbungen und Farbfehler.

In der Papierindustrie können sie zur Eliminierung von Schwermetall, insbesondere Mangan- und Eisenionen verwendet werden. Die Ablagerung von Eisen auf Papier führt zu "heißen Flecken", an denen die oxidative, katalytische Zerstörung der Zellulose beginnt.

Die beschriebenen Komplexbildner können in technischen Reinigungsmittelformulierungen für harte Oberflächen aus Metall, Kunststoff, Lack oder Glas eingesetzt werden. Als Einsatzgebiete für derartige Formulierungen kommen alkalische Entroster, alkalische Tauchentfetter, Allzweckreiniger, Autowaschmittel für Bürsten- und Hochruckwäsche, Dampfstrahlreiniger, elektrolytische Entfetter, insbesondere für Stahl, elektrolytische Entroster, elektrolytische Entzunderer, hochalkalische Reiniger, Hochdruckreiniger, Kettengleitmittel für Transportbänder von Flaschenbefüllungs- und Reinigungsanlagen, Passivierungsmittel für Stahl, Spritzentfetter und wäßrige Kaltreiniger in Betracht. Bei diesen Reinigungsmittelformulierungen kann in der Regel auf die Mitverwendung organischer Lösungsmittel verzichtet werden.

Die beschriebenen Komplexbildner können in alkalischen Reinigungsmittelformulierungen, welche im wesentlichen frei von organischen Lösungsmittel sind, für die Getränke- und Nahrungsmittelindustrie, insbesondere für die Flaschenreinigung in der Getränkeindustrie sowie die Apparatereiniging in Molkereien, in Brauereien, in der Konserven-, der Backwaren-, der Zucker-, der fettverarbeitenden und der fleischverarbeitenden Industrie, eingesetzt werden.

Die beschriebenen Komplexbildner können in Geschirreinigungsmittelformulierungen, insbesondere in phosphatfreien Mitteln für das maschinelle Geschirreinigen in Geschirrspülmaschinen im Haushalt oder in Gewerbebetrieben, z.B. Großküchen oder Restaurants, verwendet werden.

Als verschiedene Anwendungen kommen beispielsweise Anwendungen in Pharmazeutika, Kosmetika und Nahrungsmitteln in Betracht, um die metallkatalysierte Oxidation von olefinischen Doppelbindungen und damit das Ranzigwerden der Erzeugnisse zu verhindern.

In der Pflanzenernährung werden zur Behebung von Schwermetalldefiziten Kupfer-, Eisen-, Mangan- und Zink-Komplexe der neuen Komplexbildner verwendet. Die Schwermetalle werden als Chelate zugegeben, um die Ausfällung als biologisch inaktive, unlösliche Salze zu verhindern.

Weitere Anwendungsgebiete für die beschriebenen Komplexbildner sind die Rauchgaswäsche, und zwar die Entfernung von $NO_x$ aus Rauchgasen, die $H_2S$-Oxidation, die Metallextraktion, sowie Anwendungen als Katalysatoren für organische Synthesen, z.B. die Luftoxidation von Paraffinen oder die Hydroformylierung von Olefinen zu Alkoholen.

Die erfindungsgemäß verwendeten Komplexbildner für Erdalkali- und Schwermetallionen werden als Komplexbildner allgemein und speziell in Wasch- und Reinigungsmitteln sowie auch in Spül- und Waschhilfsmitteln, als Bleichmittelstabilisatoren sowie als Gerüststoffe (Builder), daneben aber auch in ihrer Funktion als reiner Komplexbildner für die genannten Kationen verwendet.

Gegenstand der Erfindung sind demnach auch die entsprechenden Wasch- und Reinigungsmittel, die diese Verbindungen neben den üblichen, den Fachmann bekannten Bestandteilen enthalten. Insbesondere sind auch Gegenstand der vorliegenden Erfindung Wasch- und Reinigungsmittel, die neben den erfindungsgemäßen Verbindungen zusätzlich ein Bleichmittelsystem, d.h. üblicherweise Bleichmittel bzw. Oxidationsmittel und Bleichaktivatoren, in der üblichen Menge enthalten, da die Wirksamkeit der erfindungsgemäßen Verbindungen in Gegenwart eines solchen Bleichmittelsystems besonders effektiv ist.

Die erfindungsgemäß verwendeten Verbindungen III bzw. IV werden in Wasch- und Reinigungsformulierungen im allgemeinen in eine Menge von 0,01 bis 50 Gew.-%, bevorzugt 0,02 bis 40 Gew.-%, insbesondere 0,03 bis 20 Gew.-%, vor allem 0,05 bis 10 Gew.-%, bezogen auf das Gesamtgewicht der Formulierung, eingesetzt.

Bei einer Verwendung bevorzugt als Gerüststoff sind Mengen von 1 bis 10 Gew.-%, bei einer Verwendung bevorzugt als Bleichmittelstabilisator für Perborate sind Mengen von 0,05 bis 1 Gew.-% besonders bevorzugt. Bei einer Verwendung insbesondere als reiner Komplexbildner in Waschmitteln sind Mengen von 0,1 bis 2 Gew.-% bevorzugt.

Die Zusammensetzung von Wasch- und Reinigungsformulierungen kann sehr unterschiedlich sein. Wasch- und Reinigungsmittel enthalten üblicherweise 2 bis 50 Gew.-% Tenside und gegebenenfalls Builder. Diese Angaben gelten sowohl für flüssige als auch für pulverförmige Wasch- und Reinigungsmittel. Beispiele für die Zusammensetzung von Waschmittelformulierungen, die in Europa, in den USA und in Japan gebräuchlich sind, findet man beispielsweise in Chemical and Engn. News, Band 67, 35 (1989) tabellarisch zusammengestellt. Weitere Angaben über die Zusammensetzung von Wasch- und Reinigungsmitteln können der WO-A 90 13581 sowie Ullmanns Encyclopädie der technischen Chemie, Verlag Chemie, Weinheim 1983, 4. Auflage, Seiten 63-160 entnommen werden. Außerdem können Waschmittelformulierungen bis zu 60 Gew.-% eines Alkalisilikates und bis zu 10 Gew.-% eines Waschmittelpolymeren enthalten. Als Alkalisilikate kommen beispielsweise die amorphen Natriumdisilikate in Betracht, die in der EP-A 0 444 415 und in der DE-A 40 04 626 beschrieben werden, sowie kristalline Schichtsilikate, die gemäß der EP-A 0 337 219 in Waschmittelformulierungen als Builder enthalten sind und gemäß der EP-B 0 164 514 zur Enthärtung von Wasser verwendet werden, und Natriumsilikate, die durch Entwassern von Natriumsilikatlösungen und Trocknen bis zu Wassergehalten von 15 bis 23, vorzugsweise 18 bis 20 Gew.-% erhältlich sind.

Waschmittel können gegebenenfalls noch ein Bleichmittel enthalten, z.B. Natriumperborat, das im Fall seines Einsatzes in Mengen bis zu 30 Gew.-% in der Waschmittelformulierung enthalten sein kann. Die Wasch- und Reinigungsmittel können gegebenenfalls weitere übliche Zusätze enthalten, z.B. Komplexbildner, Citrate, Trübungsmittel, optische Aufheller, Enzyme, Parfümöle, Farbübertragungsinhibitoren, Vergrauungsinhibitoren und/oder Bleichaktivatoren.

Wasch- und Reinigungsmittelformulierungen, die bezogen auf das Gesamtgewicht, 0,01 bis 50, bevorzugt 0,02 bis 40, insbesondere 0,03 bis 20, vor allem 0,05 bis 10 Gew.-% der erfindungsgemäß verwendeten Verbindungen enthalten, enthalten insbesondere in der Regel als zusätzliche Bestandteile, bezogen auf das Gesamtgewicht, 6 bis 25 Gew.-% Tenside, 15 bis 50 Gew.-% Builder und gegebenenfalls Co-Builder, 5 bis 35 Gew.-% Bleichmittel und gegebenenfalls Bleichmittelaktivatoren, 3 bis 30 Gew.-% Hilfsstoffe wie Enzyme, Schaumregulatoren, Korrosionsinhibitoren, optische Aufheller, Duftstoffe, Farbstoffe oder Formulierhilfsmittel, wie z.B. Natriumsulfat.

Die erfindungsgemäß verwendeten Verbindungen können in ihrer Eigenschaft als Komplexbildner, Gerüststoffe und Bleichmittelstabilisatoren auch in Wasch- und Reinigungsformulierungen zusammen mit anderen Mitteln gleicher Funktion, die aus dem Stand der Technik bekannt sind, verwendet werden, wobei die allgemeinen Eigenschaften im Hinblick auf Sequestrierung, Inkrustationsinhibierung, Vergrauungsinhibierung, Primärwaschwirkung und Bleichwirkung unter Umständen durch synergistisches Zusammenwirken deutlich verbessert werden können.

Ein solcher Synergismus tritt bei Mischungen aus

A) Hydroxamsäuren oder Hydroxamsäureethern der allgemeinen Formel III bzw. IV und

B) Polycarboxylaten in Form von

(i) Homo-, Co- oder Terpolymeren der Acryl- oder Methacrylsäure,
(ii) Homo-, Co- oder Terpolymeren der Asparaginsäure,
(iii) Homo-, Co- oder Terpolymeren von Vinylcitrat oder
(iv) niedermolekularen Mono- bis Hexacarbonsäuren

als freie Säuren oder als Alkalimetall-, Ammonium- oder substituierte Ammoniumsalze

im Gew.-Verhältnis A:B von 50:1 bis 1:20, vorzugsweise 30:1 bis 1:15, auf.

Hierbei werden Mischungen aus

A) N,N-Bis(carboxymethyl)-3-aminopropiohydroxamsäuren oder deren Ethern der allgemeinen Formel IIIa und

B) Alkalimetallsalzen der Citronensäure

im Gew.-Verhältnis A:B von 20:1 bis 1:20, insbesondere 10:1 bis 1:10, besonders bevorzugt.

Solche Mischungen sind deshalb auch Gegenstand der vorliegenden Erfindung.

Unter den genannten Polycarboxylaten sollen insbesondere verstanden werden:

(i) Homo-, Co- und Terpolymere der Acrylsäure (Methacrylsäure). Als Cokomponenten können ungesättigte Mono- und Dicarbonsäure (z.B. Crotonsäure, Maleinsäure, Itaconsäure) sowie wasserunlösliche Vinylverbindungen (z.B. Vinylacetat) in Frage kommen. Der K-Wert nach Fikentscher (1 Gew.-% in Wasser als Na-Salz) dieser Verbindung beträgt in der Regel 5 bis 100.

(ii) Homo-, Co- und Terpolymere der Asparaginsäure. Als Cokomponente können mono-, di- und polyfunktionelle, niedermolekulare und hochmolekulare Carbonsäuren (-anhydride) oder deren Salze (Na, K, Ammonium, tert. Amin) sowie wasserunlösliche Vinylverbindungen (z.B. Vinylacetat) in Frage kommen. Der K-Wert nach Fikentscher (1 Gew.-% in Wasser als Na-Salz) dieser Verbindungen beträgt in der Regel 5 bis 100.

(iii) Homo-, Co- und Terpolymere von Vinylcitrat. Als Cokomponente können mono-, di- und polyfunktionelle, niedermolekulare und hochmolekulare Carbonsäuren (-anhydride) oder deren Salze (Na, K, Ammonium, tert. Amin) sowie wasserunlösliche Vinylverbindungen (z.B. Vinylacetat) in Frage kommen. Der K-Wert nach Fikentscher (1 Gew.-% in Wasser als Na-Salz) dieser Verbindungen beträgt in der Regel 5 bis 100.

(iv) Niedermolekulare Mono-, Di-, Tri-, Tetra-, Penta- und Hexacarbonsäuren oder deren Salze. Das Grundgerüst kann aliphatischer oder aromatischer Natur sein. Beispiele sind Phthalsäure, Terephthalsäure, Benzol-hexa (oder tetra)-carbonsäure, Citronensäure, Äpfelsäure.

Übliche, dem Fachmann bekannte Bestandteile von Waschmittelformulierungen, bezogen auf die oben angegebene Rahmenvorschrift, seien im folgenden beispielsweise aufgezählt:

Geeignete Tenside sind solche, die im Molekül wenigstens einen hydrophoben organischen Rest und eine wasserlöslich machende anionische, zwitterionische oder nichtionische Gruppe enthalten. Bei dem hydrophoben Rest handelt es sich meist um einen aliphatischen Kohlenwasserstoffrest mit 8 bis 26, vorzugsweise 10 bis 22 und insbesondere 12 bis 18 C-Atomen, oder um einen alkylaromatischen Rest mit 6 bis 18, vorzugsweise 8 bis 16 aliphatischen C-Atomen.

Geeignete synthetische anionische Tenside sind insbesondere solche vom Typ der Sulfonate, Sulfate oder der synthetischen Carboxylate.

Als Tenside vom Sulfonattyp kommen Alkylbenzolsulfonate mit 4 bis 15 C-Atomen im Alkyl, Gemische aus Alken- und Hydroxyalkansulfonaten sowie Disulfonaten, wie man sie beispielsweise aus Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Weiter eignen sich Alkansulfonate, die aus Alkanen durch Sulfochlorierung oder Sulfoxydation und anschließende Hydrolyse bzw. Neutralisation bzw. durch Bisulfitaddition an Olefine erhältlich sind. Weitere brauchbare Tenside vom Sulfonattyp sind die Ester von $\alpha$-Sulfofettsäuren, z.B. die $\alpha$-Sulfofettsäuren aus hydrierten Methyl- oder Ethylestern der Kokos-, Palmkern- oder Talgfettsäure.

Geeignete Tenside vom Sulfattyp sind die Schwefelsäuremonoester primärer Alkohole, z.B. aus Kokosfettalkoholen, Talgfettalkoholen oder Oleylalkohol, und solche von sekundären Alkoholen. Weiterhin eignen sich sulfatierte Fettsäurealkanolamine, Fettsäuremonoglyceride oder Umsetzungsprodukte von 1 bis 4 Mol Ethylenoxid mit primären oder sekundären Fettalkoholen oder Alkylphenolen.

Weitere geeignete anionische Tenside sind die Fettsäureester bzw. -amide von Hydroxy- oder Aminocarbonsäuren bzw. -sulfonsäuren, wie z.B. die Fettsäuresarcoside, -glykolate, -lactate, -tauride oder -isothionate.

Die anionischen Tenside können in Form ihrer Natrium-, Kalium- und Ammoniumsalze sowie als lösliche Salze organischer Basen, wie Mono-, Di- oder Triethanolamin, vorliegen. Die üblichen Seifen, d.h. Salze der natürlichen Fettsäuren, sollen nicht unerwähnt bleiben.

Als nichtionische Tenside (Nonionics) sind z.B. Anlagerungsprodukte von 3 bis 40, vorzugsweise 4 bis 20 Mol Ethylenoxid an 1 Mol Fettalkohol, Alkylphenol, Fettsäure, Fettamin, Fettsäureamid oder Alkansulfonamid verwendbar. Besonders wichtig sind die Anlagerungsprodukte von 5 bis 16 Mol Ethylenoxid an Kokos- oder Talgfettalkohole, an Oleylalkohol oder an synthetische Alkohole mit 8 bis 18, vorzugsweise 12 bis 18 C-Atomen, sowie an Mono- oder Dialkylphenole mit 6 bis 14 C-Atomen in den Alkylresten. Neben diesen wasserlöslichen Nonionics sind aber auch nicht bzw. nicht vollständig wasserlösliche Polyglykolether mit 1 bis 4 Ethylenglykoletherresten im Molekül von Interesse, insbesondere wenn sie zusammen mit wasserlöslichen nichtionischen oder anionischen Tensiden eingesetzt werden.

Weiterhin sind als nicht ionische Tenside die wasserlöslichen, 20 bis 250 Ethylenglykolethergruppen und 10 bis 100 Propylenglykolethergruppen enthaltenden Anlagerungsprodukte von Ethylenoxid an Polypropylenglykolether, Alkylendiaminopolypropylenglykol und Alkylpolypropylenglykole mit 1 bis 10 C-Atomen in der Alkylkette brauchbar, in denen die Polypropylenglykoletherkette als hydrophober Rest fungiert.

Auch nichtionische Tenside vom Typ der Aminoxide oder Sulfoxide sind verwendbar.

Das Schaumvermögen der Tenside läßt sich durch Kombination geeigneter Tensidtypen steigern oder verringern. Eine Verringerung läßt sich ebenfalls durch Zusätze von nichttensidartigen organischen Substanzen erreichen.

Als Buildersubstanzen sind beispielsweise geeignet: Waschalkalien wie Natriumcarbonat und Natriumsilicat oder Komplexbildner wie Phosphate oder Ionenaustauscher wie Zeolithe sowie deren Mischungen. Diese Gerüst- und Aufbaustoffe haben die Aufgabe, die teils aus Wasser, teils aus Schmutz oder dem Textilgut stammenden Härteionen zu eliminieren und die Tensidwirkung zu unterstützen. Neben den genannten Buildersubstanzen können weiter im Builder sogenannte Co-Builder enthalten sein. Die Co-Builder haben in modernen Waschmitteln die Aufgabe, einige Eigenschaften der Phosphate zu übernehmen, wie z.B. Sequestrierwirkung, Schmutztragevermögen, Primär- und Sekundärwaschwirkung.

Im Builder können z.B. wasserunlösliche Silicate, wie sie beispielsweise in der DE-OS 24 12 837 beschrieben werden, und/oder Phosphate vorhanden sein. Aus der Gruppe der Phosphate können Pyrophosphat, Triphosphat, höhere Polyphosphate und Metaphosphate verwendet werden. Auch phosphorhaltige organische Komplexbildner, wie Alkanpolyphosphonsäuren, Amino- und Hydroxyalkanpolyphosphonsäuren und Phosphonocarbonsäuren kommen als weitere Waschmittelinhaltsstoffe in Betracht. Beispiele solcher Waschmitteladditive sind z.B. die folgenden Verbindungen: Methan-diphosphonsäure, Propan-1,2,3-triphosphonsäure, Butan-1,2,3,4-tetraphosphonsäure, Polyvinylphosphonsäure, 1-Aminoethan-1,1-diphosphonsäure, 1-Amino-1-phenyl-1,1-diphosphonsäure, Aminotris-methylen-triphosphonsäure, Methylamino- oder Ethylaminobismethylen-diphosphonsäure, Ethylendiaminotetramethylentetraphosphonsäure, Diethylentriaminopentamethylenpentaphosphonsäure, 1-Hydroxyethan-1,1-diphonsphonsäure, Phosphonoessig- und Phosphonopropionsäure, Mischpolymerisate aus Vinylphosphonsäure und Acryl- und/oder Maleinsäure sowie deren teil- oder vollneutralisierten Salze.

Weitere organische Verbindungen, die als Komplexierungsmittel für Calcium wirken und in Waschmittelformulierungen enthalten sein können, sind Polycarbonsäuren, Hydroxycarbonsäuren und Aminocarbonsäuren, die meist in Form ihrer wasserlöslichen Salze eingesetzt werden.

Beispiele für Polycarbonsäuren sind Dicarbonsäuren der allgemeinen Formel $HOOC-(CH_2)_m-COOH$ mit m = 0 bis 8, außerdem Maleinsäure, Methylenmalonsäure, Citraconsäure, Mesaconsäure, Itaconsäure, nicht cyclische Polycarbonsäuren mit wenigstens 3 Carboxylgruppen im Molekül, wie z.B. Tricarballylsäure, Aconitsäure, Ethylentetracarbonsäure, 1,1,3-Propan-tetracarbonsäure, 1,1,3,3,5,5-Pentan-hexacarbonsäure. Hexanhexacarbonsäure, cyclische Di- oder Polycarbonsäuren, wie z.B. Cyclopentan-tetracarbonsäure, Cyclohexan-hexacarbonsäure, Tetrahydrofurantetracarbonsäure, Phthalsäure, Terephthalsäure, Benzoltri-, -tetra- oder -pentacarbonsäure sowie Mellithsäure.

Beispiele für Hydroxymono- oder -polycarbonsäuren sind Glykolsäure, Milchsäure, Äpfelsäure, Tartronsäure, Methyltartronsäure, Gluconsäure, Glycerinsäure, Citronensäure, Weinsäure oder Salicylsäure.

Beispiele für Aminocarbonsäuren sind Glycin, Glycylglycin, Alanin, Asparagin, Glutaminsäure, Aminobenzoesäure, Iminodi- oder -triessigsäure, Hydroxyethyliminodiessigsäure, Ethylendiaminotetraessigsäure, Hydroxyethyl-ethylendiamin-triessigsäure, Diethylentriamin-pentaessigsäure sowie höhere Homologe, die durch Polymerisation eines N-Azidirylcarbonsäurederivates, z.B. der Essigsäure, Bernsteinsäure, Tricarballylsäure und anschließende Verseifung, oder durch Kondensation von Polyaminen mit einem Molekulargewicht von 500 bis 10.000 mit chloressigsauren oder bromessigsauren Salzen hergestellt werden können.

Als Co-Buildersubstanzen werden bevorzugt polymere Carbonsäuren verwendet. Zu diesen polymeren Carbonsäuren sind die Carboxymethylether der Zucker, der Stärke und der Cellulose zu zählen.

Unter den polymeren Carbonsäuren spielen z.B. die Polymerisate der Acrylsäure, Maleinsäure, Itaconsäure, Mesaconsäure, Aconitsäure, Methylenmalonsäure und Citraconsäure, die Copolymerisate der oben genannten Carbonsäuren untereinander, z.B. ein Copolymerisat aus Acrylsäure und Maleinsäure im Verhältnis 70:30 vom Molgewicht 70.000, oder mit ethylenisch ungesättigten Verbindungen wie Ethylen, Propylen, Isobutylen, Vinylalkohol, Vinylmethylether, Furan, Acrolein, Vinylacetat, Acrylamid, Acrylnitril, Methacrylsäure oder Crotonsäure, z.B. die 1:1-Mischpolymerisate aus Maleinsäureanhydrid und Methylvinylether vom Molgewicht 70.000 oder die Mischpolymerisate von Maleinsäureanhydrid und Ethylen bzw. Propylen bzw. Furan, eine besondere Rolle.

In den Co-Buildern können weiterhin Schmutzträger enthalten sein, die den von der Faser abgelösten Schmutz in der Flotte suspendiert halten und so das Vergrauen inhibieren. Hierzu sind wasserlösliche Kolloide meist organischer Natur geeignet, wie beispielsweise die wasserlöslichen Salze polymerer Carbonsäuren, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich lösliche Stärkepräparate und andere als die oben genannten Stärkeprodukte verwenden, z.B. abgebaute Stärke oder Aldehydstärken; auch Polyvinylpyrrolidon ist für diesen Zweck brauchbar.

Bleichmittel sind insbesondere Wasserstoffperoxid und Derivate hiervon oder aktivchlorliefernde Verbindungen. Unter den als Bleichmittel dienenden, in Wasser $H_2O_2$-liefernden Verbindungen haben Natriumperborat-Hydrate wie $NaBO_2.H_2O_2.3H_2O$ und $NaBO_2.H_2O_2$ besondere Bedeutung. Es sind aber auch andere $H_2O_2$ liefernde Borate brauchbar. Diese Verbindungen können teilweise oder vollständig durch andere Aktivsauerstoffträger, insbesondere durch Peroxyhydrate, wie Peroxycarbonate, Peroxyphosphonate, Citratperhydrate, Harnstoff-$H_2O_2$- oder Melamin-$H_2O_2$-Verbindungen sowie $H_2O_2$ liefernde persaure Salze, wie z.B. Caroate, Perbenzoate oder Peroxyphthalate, ersetzt werden.

Es können neben den erfindungsgemäßen übliche wasserlösliche und/oder wasserunlösliche Stabilisatoren für die Peroxyverbindungen zusammen mit diesen in Mengen von 0,25 bis 10 Gew.-%, bezogen auf die Peroxyverbindung, eingearbeitet werden. Als wasserunlösliche Stabilisatoren eignen sich die meist durch Fällung aus wäßrigen Lösungen erhaltenen Magnesiumsilikate $MgO:SiO_2$ der Zusammensetzung 4:1 bis 1:4, vorzugsweise 2:1 bis 1:2 und insbesondere 1:1. An deren Stelle sind auch andere Erdalkalimetallsilikate entsprechender Zusammensetzung brauchbar.

Um beim Waschen bereits bei Temperaturen unterhalb 80°C, insbesondere im Bereich von 40 bis 60°C, eine befriedigende Bleichwirkung zu erreichen, werden zweckmäßigerweise Bleichaktivatoren in die Waschmittel eingearbeitet, vorteilhaft in einer Menge von, bezogen auf die $H_2O_2$-liefernde Verbindung, 5 bis 30 Gew.-%.

Als Aktivatoren für in Wasser $H_2O_2$-liefernde Perverbindungen dienen bestimmte, mit $H_2O$, organische Persäuren bildende N-Acyl oder O-Acyl-Verbindungen, insbesondere Acetyl-, Propionyl- oder Benzoylverbindungen, sowie Kohlensäure- bzw. Pyrokohlensäureester. Brauchbare Verbindungen sind unter anderem:

- Zuckerester, z.B. Pentaacetylglucose;
- Acyloxybenzolsulfonsäuren und deren Alkali- und Erdalkalimetallsalze, z.B. Natrium-p-isononanoyloxy-benzolsulfonat oder Natrium-p-benzoyloxy-benzolsulfonat;
- N-diacylierte und N,N'-tetraacylierte Amine, z.B. N,N,N',N'-Tetraacetyl-methylendiamin und -ethylendiamin, N,N-Diacetylanilin, N,N-Diacetyl-p-toluidin oder 1,3-diacylierte Hydantoine wie 1,3-Diacetyl-5,5-dimethylhydantoin;
- Benz-(4H)1,3-oxazin-4-one, z.B. 2-Phenyl-benz-(4H)1,3-oxazin-4-on oder 2-Methyl-benz-(4H)1,3-oxazin-4-on;
- N-Alkyl-N-sulfonyl-carbonamide, z.B. N-Methyl-N-mesyl-acetamid oder N-Methyl-N-mesyl-benzamid;
- N-acylierte cyclische Hydrazide, acylierte Triazole oder Urazole, z.B. Monoacetyl-maleinsäurehydrazid;
- O,N,N-trisubstituierte Hydroxylamine, z.B O-Benzoyl-N,N-succinyl-hydroxylamin, O-Acetyl-N,N-succinyl-hydroxylamin oder O,N,N-Triacetylhydroxylamin;
- N,N'-Diacyl-sulfurylamide, z.B. N,N'-Dimethyl-N,N'-diacetylsulfurylamid oder N,N'-Diethyl-N,N'-dipropionyl-sulfurylamid;
- Triacylcyanurate, z.B. Triacetylcyanurat oder Tribenzoylcyanurat;
- Carbonsäureanhydride, z.B. Benzoesäureanhydrid, m-Chlorbenzoesäureanhydrid oder Phthalsäureanhydrid;
- 1,3-Diacyl-4,5-diacyloxy-imidazole, z.B. 1,3-Diacetyl-4,5--diacetoxyimidazolin;
- Tetraacetylglycoluril und Tetrapropionylglycoluril;

- diacylierte 2,5-Diketopiperazine, z.B. 1,4-Diacetyl-2,5-diketopiperazin;

- Acylierungsprodukte von Propylendiharnstoff und 2,2-Dimethylpropylendiharnstoff, z.B. Tetraacetylpropylendiharnstoff;

- $\alpha$-Acyloxy-polyacyl-malonamide, z.B. $\alpha$-Acetoxy-N,N'-diacetylmalonamid;

- Diacyl-dioxohexahydro-1,3,5-triazine, z.B. 1,5-Diacetyl-2,4-dioxohexahydro-1,3,5-triazin.

Als Bleichmittel können auch Aktivchlorverbindungen anorganischer oder organischer Natur eingesetzt werden. Zu den anorganischen Aktivchlorverbindungen gehören Alkalihypochlorite, die insbesondere in Form ihrer Mischsalze bzw. Anlagerungsverbindungen an Orthophosphate oder kondensierte Phosphate, wie beispielsweise an Pyro- und Polyphosphate oder an Alkalisilikate, verwendet werden können. Enthalten die Wasch- und Waschhilfsmittel Monopersulfate und Chloride, so bildet sich in wäßriger Lösung Aktivchlor.

Als organische Aktivchlorverbindungen kommen insbesondere die N-Chlorverbindungen in Frage, bei denen ein oder zwei Chloratome an ein Stickstoffatom gebunden sind, wobei vorzugsweise die Dritte Valenz der Stickstoffatome an eine negative Gruppe führt, insbesondere an eine CO- oder $SO_2$-Gruppe. Zu diesen Verbindungen gehören Dichlor- und Trichlorcyanursäure bzw. deren Salze, chlorierte Alkylguanide oder Alkylbiguanide, chlorierte Hydantoine und chlorierte Melamine.

Als Schaumregulatoren eignen sich, vor allem bei der Verwendung von Tensiden vom Sulfonat- oder Sulfattyp, kapillaraktive Carboxy- oder Sulfobetaine sowie die oben erwähnten Nonionics vom Alkylolamidtyp. Für diesen Zweck sind auch Fettalkohole oder höhere endständige Diole geeignet.

Ein verringertes Schäumvermögen, das vor allem beim maschinellen Waschen erwünscht ist, erreicht man vielfach durch Kombination verschiedener Tensidtypen, z.B. von Sulfaten und/oder Sulfonaten mit Nonionics und/oder mit Seifen. Bei Seifen steigt die Schaumdämpfung mit dem Sättigungsgrad und der C-Zahl des Fettsäureesters an; Seifen der gesättigten $C_{20}$-$C_{24}$-Fettsäuren eignen sich deshalb besonders gut als Schaumdämpfer.

Zu den nichttensidartigen Schauminhibitoren gehören gegebenenfalls Chlor enthaltende N-alkylierte Aminotriazine, die man durch Umsetzen von 1 Mol Cyanurchlorid mit 2 bis 3 Mol eines Mono- und/oder Dialkylamins mit 6 bis 20, vorzugsweise 8 bis 18 C-Atomen im Alkylrest erhält. Ähnlich wirken propoxylierte und/oder butoxylierte Aminotriazine, z.B. Produkte, die man durch Anlagern von 5 bis 10 Mol Propylenoxid an 1 Mol Melamin und weiteres Anlagern von 10 bis 50 Mol Butylenoxid an dieses Propylenoxidderivat erhält.

Ebenfalls geeignet als nichttensidartige Schauminhibitoren sind wasserunlösliche organische Verbindungen, wie Paraffine oder Halogenparaffine mit Schmelzpunkten unterhalb von 100°C, aliphatische $C_{18}$- bis $C_{40}$-Ketone sowie aliphatische Carbonsäureester, die im Säure- oder im Alkoholrest, gegebenenfalls auch in jedem dieser beiden Reste, wenigstens 18 C-Atome enthalten, z.B. Triglyceride oder Fettsäurefettalkoholester; sie lassen sich vor allem bei Kombinationen von Tensiden des Sulfat- und/oder Sulfonattyps mit Seifen zum Dämpfen des Schaumes verwenden.

Die Waschmittel können optische Aufheller für Baumwolle, für Polyamid-, Polyacrylnitril- oder Polyestergewebe enthalten. Als optische Aufheller sind beispielsweise Derivate der Diaminostilbendisulfonsäure für Baumwolle, Derivate von 1,3-Diarylpyrazolinen für Polyamid, quartäre Salze von 7-Methoxy-2-benzimidazolyl-(2')-benzofuran oder von Derviaten aus der Verbindungsklasse der 7-[1',2',5'-Triazolyl-(1')]-3-[1",2",4"-triazolyl-(1")]-cumarine für Polyacrylnitril geeignet. Für Polyester geeignete Aufheller sind beispielsweise Produkte aus der Verbindungsklasse der substituierten Styrile, Ethylene, Thiophene, Naphthalindicarbonsäuren oder Derivate davon, Stilbene, Cumarine und Naphthalimide.

Als weitere Hilfsstoffe oder Formulierungsmittel können die dem Fachmann an sich bekannten Stoffe verwendet werden, z.B. Lösungsvermittler wie Xylol- oder Cumolsulfonate, Stellmittel wie Natriumsulfat, Enzyme oder Parfümöle.

Die erfindungsgemäßen Wasch- und Reinigungsmittel können pulverförmig oder flüssig sein.

Herstellungsbeispiele

Beispiel 1

Synthese von N,N-Bis(carboxymethyl)-3-aminopropiohydroxamsäure-Mononatriumsalz (Einstufenvariante)

66,5 g (0,50 mol) Iminodiessigsäure wurden in 200 ml Wasser gelöst und mit 40,0 g (0,50 mol) 50 gew.-%iger wäßriger NaOH auf pH 8 gebracht. Zu dieser Lösung tropfte man dann bei 20°C innerhalb von 30 min 43,0 g (0,50 mol) Acrylsäuremethylester. Die Lösung wurde dann für 12 h bei 20°C gerührt, nach dieser Zeit betrug der Umsetzungsgrad laut HPLC 95 %. Anschließend wurden 34,3 g (0,50 mol) Hydroxylamin-Hydrochlorid zugegeben und die Mischung für weitere 3 h bei 20°C gerührt, wobei durch Zugabe von insgesamt 40,0 g (0,50 mol) 50 gew.-%iger wäßriger NaOH auf pH 7 konstant gehalten wurde. Nach dieser Zeit war das Zwischenprodukt N,N-Bis(carboxymethyl)-3-aminopropionsäuremethylester abreagiert und es ließ sich nur noch die Titelverbindung als Endprodukt nachweisen; das Calcium-Komplexier-Vermögen entsprach 94 % der Theorie. Durch Zusatz von 650 ml Methanol fiel die Titelverbindung als hygroskopisches Öl in einer Ausbeute von 106 g (entsprechend 97 % der Theorie) aus.

Beispiel 2

Synthese von N,N-Bis(carboxymethyl)-3-aminopropiohydroxamsäure-Dinatriumsalz (Zweistufenvariante)

Beispiel 2a

Herstellung von N,N-Bis(carboxymethyl)-3-aminopropionsäuremethylester

133 g (1,00 mol) Iminodiessigsäure wurden in 530 ml Wasser gelöst und die Lösung wurde mit 40,5 g (1,00 mol) festem NaOH auf pH 7,0 eingestellt. Zu dieser Lösung wurden 86,0 g (1,00 mol) Acrylsäuremethylester bei 20°C getropft, danach wurde 6 h nachgerührt. Der Acrylsäuremethylester-Gehalt war nach dieser Zeit auf 0,6 % der Ausgangsmenge abgesunken, der pH-Wert wurde durch Zugabe von 98,5 ml konz. HCl auf 2 eingestellt. Über Nacht fiel bei 5°C N,N-Bis(carboxymethyl)-3-aminopropionsäuremethylester als farbloser Niederschlag aus. Nach Abfiltrieren und Trocknen resultierten 210 g (entsprechend einer Ausbeute von 96 % der Theorie) eines reinen Produktes vom Schmelzpunkt 141°C.

Beispiel 2b

Umsetzung zu N,N-Bis(carboxymethyl)-3-aminopropiohydroxamsäure-Dinatriumsalz

51,5 g (0,235 mol) des Esters aus Beispiel 2a wurden in 50 ml Methanol suspendiert und zu dieser Suspension bei 20°C 600 g (1,26 mol) einer 6,9 gew.-%igen methanolischen Hydroxylamin-Lösung getropft. Die Reaktionsmischung wurde 25 h bei 20°C weitergerührt und anschließend durch Abdestillieren von flüchtigen Bestandteilen befreit. Das verbleibende harzige Produkt war stark hygroskopisch und wurde deshalb in 130 ml Wasser gelöst und mit 94,0 g (0,470 mol) 20 gew.-%iger wäßriger NaOH versetzt, erneut vom Lösungsmittel befreit und im Vakuum getrocknet. Das farblose Pulver wurde durch zweimaliges Umfällen aus einem Methanol/Wasser/Aceton-Gemisch rein erhalten. Es resultierten 58,5 g (entsprechend einer Ausbeute von 92 % der Theorie) der Titelverbindung.

Beispiel 3

N,N-Bis(carboxymethyl)-3-aminopropio-N'-methyl-hydroxamsäure-Dinatriumsalz aus Iminodiessigsäure

Zu einer Suspension von 30,6 g Iminodiessigsäure in 95 g Wasser wurden nacheinander 36,8 g 50 gew.-%ige wäßrige Natronlauge und 19,8 g Acrylsäuremethylester bei 15°C gegeben. Nach 45 min bei 15°C wurden zu der Lösung 19,8 g N-Methyl-hydroxylammoniumchlorid gelöst in 50 g Wasser zugetropft und gleichzeitig der pH-Wert durch Zugabe von insgesamt 17,8 g 50 gew.-%iger wäßriger Natronlauge bei 9,8 gehalten. Nach 30 h bei 20°C war der Umsatz an Ester 99 %, durch Gefriertrocknung wurden 80,0 g Produkt mit einem Gehalt von 57 % N,N-Bis(carboxymethyl)-3-aminopropio-N'-methylhydroxamsäure-Dinatriumsalz (entsprechend 71 % Ausbeute der Theorie), 7,5 % Wasser, 16 % Kochsalz und 19 % β-Alanin-N,N-diessigsäure-Dinatriumsalz erhalten.

Beispiel 4

N,N-Bis(carboxymethyl)-3-aminopropio-hydroxamsäure-O-methylether-Dinatriumsalz aus N,N-Bis(carboxymethyl)-3-aminopropionsäuremethylester

Zu einer Suspension von 13,3 g Iminodiessigsäure in 41,5 g Wasser wurden nacheinander 16,0 g 50 gew.-%ige wäßrige Natronlauge und 8,6 g Acrylsäuremethylester bei 15°C gegeben. Nach 60 min bei 10°C wurden zu der Lösung 8,3 g Methoxyamin-Hydrochlorid zugegeben und gleichzeitig der pH-Wert durch Zugabe von insgesamt 8,4 g 50 gew.-%iger Natronlauge bei 10,0 gehalten. Nach 30 h bei 20°C war der Umsatz an Ester 99 % der Theorie, durch Gefriertrocknung wurden 31,1 g Produkt mit einem Gehalt von 68 % N,N-Bis(carboxymethyl)-3-aminopropio-hydroxamsäure-O-methylether-Dinatriumsalz und einem Calciumbindevermögen von 2,435 mmol/g (entsprechend 77 % Ausbeute der Theorie), 5,6 % Wasser, 19 % Kochsalz erhalten.

Beispiel 5

D,L-Asparaginsäure-N-propiohydroxamsäure aus D,L-Asparaginsäure

Zu einer Suspension von 66,5 g Asparaginsäure in 207 g Wasser wurden nacheinander 80 g 50 gew.-%ige wäßrige Natronlauge und 43,0 g Acrylsäuremethylester bei 10°C zugetropft. Nach 4 h bei 10°C war der Acrylsäuremethylester zu 99 % der Theorie abreagiert und es wurden 33,0 g Hydroxylammoniumchlorid gelöst in 50 g Wasser zugetropft. Dabei wurde der pH-Wert durch Zugabe von insgesamt 38,6 g 50 gew.-%iger wäßriger Natronlauge bei 11 gehalten. Nach 30 h bei Raumtemperatur wurde durch Zugabe von 34 g 37 gew.-%igem wäßrigem Chlorwasserstoff der pH-Wert auf 2 gesenkt und durch Abdestillation von Wasser aufkonzentriert, dadurch erhielt man 340 g einer Lösung von 28 % D,L-Asparaginsäure-N-propiohydroxamsäure (entsprechend 92,5 % der theoretischen Ausbeute).

Beispiel 6

N,N-Bis(carboxymethyl)-3-aminopropiohydroxamsäure-Dinatriumsalz aus Iminodiessigsäure und Acrylamid

Zu einer Suspension von 13,8 g Iminodiessigsäure in 60 g Wasser wurden nacheinander 16,8 g 50 gew.-%ige wäßrige Natronlauge und 7,4 g Acrylsäureamid gegeben und 4 h bei Raumtemperatur gerührt. Zu der entstandenen Lösung von 26 Gew.-% N,N-Bis(carboxymethyl)propionsäureamid-Dinatriumsalz wurde dann 6,3 g Hydroxylammoniumchlorid gegeben und gleichzeitig wurde der pH-Wert durch Zugabe von insgesamt 6,8 g 50 gew.-%iger wäßriger Natronlauge bei 10 gehalten. Nach 21 h bei 20°C erhielt man eine Lösung von 20,8 % N,N-Bis(carboxymethyl)-3-aminopropiohydroxamsäure-Dinatriumsalz mit einem Eisenbindevermögen von 0,788 mmol/g (entsprechend einer Ausbeute von 97 % der Theorie).

Beispiel 7

N,N-Bis(carboxymethyl)-3-amino-2-hydroxy-propiohydroxamsäure-Dinatriumsalz aus N,N-Bis(carboxymethyl)-3-amino-2-hydroxypropionsäureamid-Dinatriumsalz

Zu eine 17 gew.-%igen wäßrigen Lösung von 142 g N,N-Bis(carboxymethyl)-3-amino-2-hydroxy-propionsäureamid-Dinatriumsalz wurden nacheinander 3,4 g 50 gew.-%ige wäßrige Natronlauge und 6,3 g Hydroxylammoniumchlorid addiert. Dabei wurde der pH-Wert durch Zugabe von insgesamt 7,8 g 50 gew.-%iger wäßriger Natronlauge bei 10 gehalten. Nach 30 h bei 40°C erhielt man 159 g einer Lösung von 11,8 % N,N-Bis(carboxymethyl)-3-amino-2-hydroxypropiohydroxamsäure-Dinatriumsalz mit einem Eisenbindevermögen von 0,458 mmol/g (entsprechend einer Ausbeute von 73 % der Theorie).

Beispiel 14

Abbauuntersuchungen von N,N-Bis(carboxymethyl)-3-aminopropiohydroxamsäure-Dinatriumsalz aus Beispiel 2

Standversuch nach Zahn-Wellens (nach EG-Richtlinie 88/302/EWG, OECD 302 B, ISO 9888):
DOC-Eliminationsgrad nach 17 Tagen 86 % (Kontrollsubstanz Ethylenglykol: DOC 98 %)
Modifizierter Sturmtest (nach 84/449/EWG, OECD 301 B, ISO 9439):
DOC-Eliminationsgrad nach 35 Tagen 100 %, $CO_2$-Entwicklung 72 % der Theorie

Anwendungstechnische Eigenschaften

Die Substanz aus Beispiel 1 wurde als Textil-Waschmitteladditiv in ihrer Funktion als Cobuilder (Inkrustationsinhibitor) getestet. Dazu wurde sie in eine Waschmittelformulierung A der folgenden Zusammensetzung eingearbeitet:

| | |
|---|---|
| 6,25 Gew.-Teile | Natrium-Dodecylbenzolsulfonat |
| 4,70 Gew.-Teile | eines mit 7 mol Ethylenoxid umgesetzten $C_{13}/C_{15}$-Oxoalkohols |
| 1,25 Gew.-Teile | Magnesiumsilicat |
| 10,00 Gew.-Teile | wasserfreies Natriumcarbonat |
| 6,00 Gew.-Teile | Natriummetasilicat-Tetrahydrat |
| 20,00 Gew.-Teile | Natriumperborat-Tetrahydrat |
| 6,75 Gew.-Teile | wasserfreies Natriumsulfat |
| 2,80 Gew.-Teile | Seife |

| | |
|---|---|
| 0,60 Gew.-Teile | Natrium-Carboxymethylcellulose |
| 30,00 Gew.-Teile | Zeolith A |
| 5,00 Gew.-Teile | N,N-Bis(carboxymethyl)-3-aminopropiohydraxamsäure-Mononatriumsalz aus Beispiel 1 |
| Rest auf 100 Gew.-Teile | Wasser |

Mit der Waschmittelformulierung A wurden Testgewebe aus Baumwolle gewaschen. Nach dem Waschprozeß wurde der Aschegehalt des Gewebes ermittelt, indem 5 g des Testgewebes 2 Stunden bei 700°C verascht wurden.

Die Wirkung (W) des Waschmitteladditivs in der Formulierung A wird in Prozent Wirksamkeit angegeben, wobei 0 % Wirkung dem Aschegehalt ohne Inkrustationsinhibitor, d.h. ohne Waschmitteladditiv (A-ohne) entspricht und 100 % Wirkung dem Aschegehalt des Gewebes vor dem Waschen (A-Null).

Die Wirkung W des Additivs wird nach folgendem Ansatz aus dem ermittelten Aschegehälten (A-Additiv) berechnet:

$$W = \left(1 - \frac{\text{(A-Additiv) - (A-Null)}}{\text{(A-ohne) - (A-Null)}}\right) \times 100\ [\%]$$

Es wurden folgende Waschbedingungen gewählt:

| | |
|---|---|
| Gerät: | Launder-O-meter der Fa. Atlas |
| Zahl der Waschzyklen: | 15 |
| Waschflotte: | 250 g |
| Wasserhärte: | 4 mmol pro Liter (Ca:Mg = 4:1) |
| Waschdauer: | 30 min bei 60°C (einschließlich Aufheizzeit) |
| Flottenverhältnis: | 1:12,5 |
| Prüfgewebe: | Baumwollnesselgewebe |

Der Aschegehalt des Baumwollgewebes vor dem Waschen (A-Null) betrug 0,04 Gew.-%, der maximale Aschegehalt ohne Inkrustationsinhibitor (A-ohne), 6,91 Gew.-%. Mit der Substanz aus Beispiel 1 wurde ein Aschegehalt von 1,02 Gew.-% ermittelt, was gemäß obiger Gleichung einer Wirkung W von 85,7 % entspricht.

Ein in der gleichen Waschserie analog durchgeführter Vergleichsversuch mit der gleichen Menge Citronensäure-Trinatriumsalz als aus dem Stand der Technik bekanntem Inkrustationsinhibitor alleine ohne erfindungsgemäße Hydroxamsäuren lieferte eine Wirkung W von 38,8 %.

Bei Verwendung einer Mischung aus der Hydroxamsäure aus Beispiel 1 und Citronensäure-Trinatriumsalz ließ sich die inkrustationsinhibierende Wirkung aufgrund des vorliegenden synergistischen Effektes noch steigern. Inkrustationsbestimmungen ergaben bei Verwendung einer entsprechenden Mischung im Gew.-Verhältnis 1:1 bei einer Konzentration von jeweils 1,6 Gew.-% Hydroxamsäure und Trinatriumcitrat in einer zu A analogen Waschmittelformulierung eine experimentell gemessene Wirkung W von 54,4 %, wogegen die rechnerisch ermittelte Wirkung W als Summe für beide Einzelkomponenten nur 47 % betrug (8,6 % für Trinatriumcitrat + 38,4 % für die Hydroxamsäure).

## Patentansprüche

1. Verwendung von Hydroxamsäuren und Hydroxamsäureethern der allgemeinen Formel III und IV

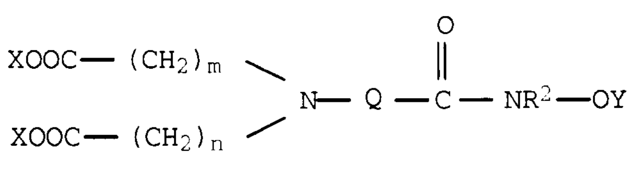

(III)

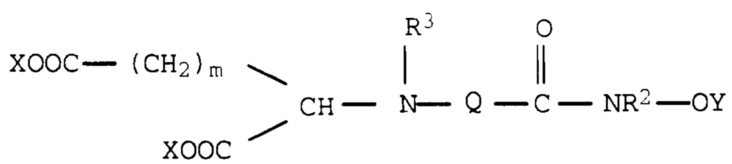

(IV)

in denen

$R^2$ Wasserstoff oder $C_1$- bis $C_{18}$-Alkyl bezeichnet,

$R^3$ Wasserstoff oder eine Gruppierung der Formel $-Q-CO-NR^2-OY$ bedeutet,

Q für eine $C_1$- bis $C_8$-Alkylengruppe steht, welche zusätzlich bis zu 8 Hydroxylgruppen tragen kann,

m und n jeweils für die Zahl 1 oder 2 stehen,

X Wasserstoff, Alkalimetall, Ammonium oder substituiertes Ammonium bezeichnet und

Y Wasserstoff, Alkalimetall, Ammonium, substituiertes Ammonium oder $C_1$- bis $C_{18}$-Alkyl bedeutet,

als Gerüststoff und Bleichstabilisatoren in Wasch- und Reinigungsmitteln.

2. Verwendung nach Anspruch 1 von N,N-Bis(carboxymethyl)-3-aminopropiohydroxamsäuren und deren Ethern der allgemeinen Formel IIIa

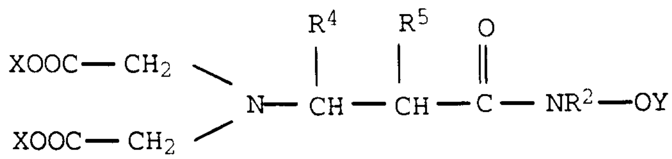

(IIIa)

in der

$R^4$ für Wasserstoff, Methyl oder Ethyl steht,

$R^5$ für Wasserstoff, Methyl, Ethyl oder Hydroxyl steht und

$R^2$, X und Y die oben genannten Bedeutungen haben.

3. Wasch- und Reinigungsmittel, enthaltend eine oder mehrere Hydroxamsäuren oder Hydroxamsäureether gemäß Anspruch 1 oder 2 in einer Menge von 0,01 bis 50 Gew.-%, bezogen auf das Gesamtgewicht.

4. Wasch- und Reinigungsmittel nach Anspruch 3, enthaltend zusätzlich ein Bleichmittelsystem in der üblichen Menge.

5. Hydroxamsäuren und Hydroxamsäureether der allgemeinen Formel III und IV

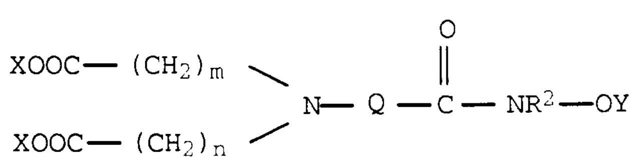

(III)

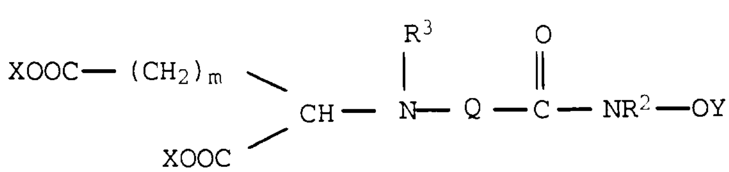

(IV)

in denen

$R^2$ Wasserstoff oder $C_1$- bis $C_{18}$-Alkyl bezeichnet,

$R^3$ Wasserstoff oder eine Gruppierung der Formel -Q-CO-$NR^2$-OY bedeutet,

Q für eine $C_1$- bis $C_8$-Alkylengruppe steht, welche zusätzlich bis zu 8 Hydroxylgruppen tragen kann,

m und n jeweils für die Zahl 1 oder 2 stehen,

X Wasserstoff, Alkalimetall, Ammonium oder substituiertes Ammonium bezeichnet und

Y Wasserstoff, Alkalimetall, Ammonium, substituiertes Ammonium oder $C_1$- bis $C_{18}$-Alkyl bedeutet,

mit Ausnahme von N,N-Bis(carboxymethyl)-2-aminoacethydroxamsäure, N-Carboxymethyl-N-(β-carboxyethyl)-2-aminoacethydroxamsäure und N,N-Bis(carboxymethyl)-3-aminopropiohydroxamsäure.

6. N,N-Bis(carboxymethyl)-3-aminopropiohydroxamsäuren und deren Ether der allgemeinen Formel IIIa

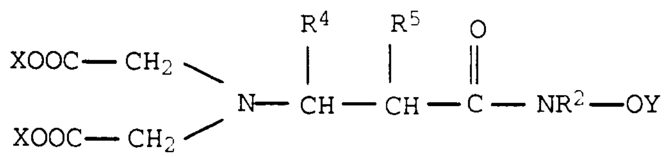

(IIIa)

in der

$R^4$ für Wasserstoff, Methyl oder Ethyl steht,

$R^5$ für Wasserstoff, Methyl, Ethyl oder Hydroxyl steht,

$R^2$ Wasserstoff oder $C_1$- bis $C_{18}$-Alkyl bezeichnet,

X Wasserstoff, Alkalimetall, Ammonium oder substituiertes Ammonium bezeichnet und

Y Wasserstoff, Alkalimetall, Ammonium, substituiertes Ammonium oder $C_1$- bis $C_{18}$-Alkyl bedeutet,

mit Ausnahme von N,N-Bis(carboxymethyl)-3-aminopropiohydroxamsäure.

7. Verfahren zur Herstellung von N,N-Bis(carboxymethyl)-3-aminopropiohydroxamsäuren und deren Ethern IIIa gemäß Anspruch 2, dadurch gekennzeichnet, daß man Iminodiessigsäure der Formel VI

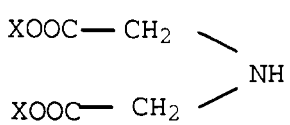

(VI)

mit einem α,β-ungesättigten Carbonsäureester der allgemeinen Formel VII

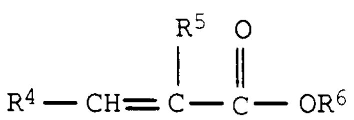

(VII)

oder einem α,β-ungesättigten Carbonsäureamid der allgemeinen Formel VIII

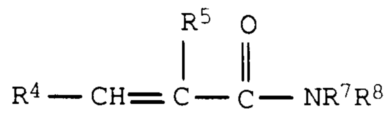

(VIII)

zum N,N-Bis(carboxymethyl)-3-aminopropionsäureester der allgemeinen Formel IX

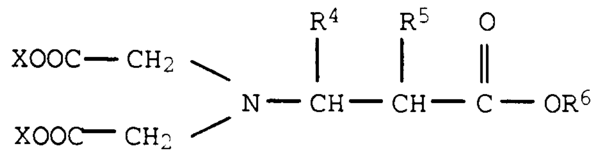

(IX)

bzw. zum N,N-Bis(carboxymethyl)-3-aminopropionsäureamid der allgemeinen Formel X

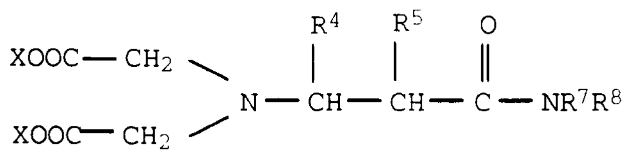

(X)

umsetzt und anschließend aus (IX) bzw. (X) durch Umsetzung mit einem Hydroxylamin oder einem Hydroxylamin-$C_1$- bis $C_{18}$-alkylether das Endprodukt IIIa herstellt, welches gewünschtenfalls durch Behandlung mit den X bzw. Y zugrundeliegenden Basen in die Salzform übergeführt werden kann, wobei die Variablen $R^4$, $R^5$, X und Y die oben genannten Bedeutungen haben sowie $R^6$ $C_1$- bis $C_4$-Alkyl bedeutet und $R^7$ und $R^8$ Wasserstoff oder $C_1$- bis $C_4$-Alkyl bezeichnen.

**8.** Mischungen aus

A) Hydroxamsäuren oder Hydroxamsäureethern gemäß Anspruch 1 und

B) Polycarboxylaten in Form von

(i) Homo-, Co- oder Terpolymeren der Acryl- oder Methacrylsäure,
(ii) Homo-, Co- oder Terpolymeren der Asparaginsäure,
(iii) Homo-, Co- oder Terpolymeren von Vinylcitrat oder
(iv) niedermolekularen Mono- bis Hexacarbonsäuren

als freie Säuren oder als Alkalimetall-, Ammonium- oder substituierte Ammoniumsalze

im Gew.-Verhältnis A:B von 50:1 bis 1:20.

**9.** Mischungen aus

A) N,N-Bis(carboxymethyl)-3-aminopropiohydroxamsäuren oder deren Ethern der allgemeinen Formel IIIa gemäß Anspruch 2 und

B) Alkalimetallsalzen der Citronensäure

im Gew.-Verhältnis A:B von 20:1 bis 1:20.

## Claims

**1.** The use of hydroxamic acids and hydroxamic acid ethers of the general formula III and IV

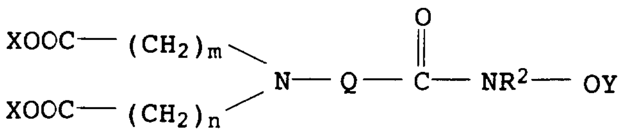

(III)

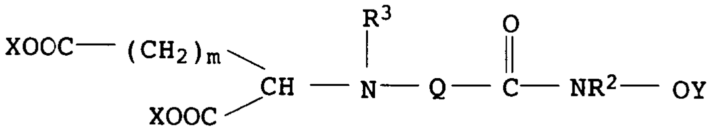

(IV)

where

$R^2$ is hydrogen or $C_1$-$C_{18}$-alkyl,

$R^3$ is hydrogen or a group of the formula $—Q—CO—NR^2—OY$,

Q is a $C_1$-$C_8$-alkylene group which can additionally carry up to 8 hydroxyl groups,

m and n are each 1 or 2,

X is hydrogen, alkali metal, ammonium or substituted ammonium, and

Y is hydrogen, alkali metal, ammonium, substituted ammonium or $C_1$-$C_{18}$-alkyl,

as builder and bleach stabilizers in detergents and cleaners.

**2.** The use as claimed in claim 1 of N,N-bis(carboxymethyl)-3-aminopropiohydroxamic acids and their ethers of the general formula IIIa

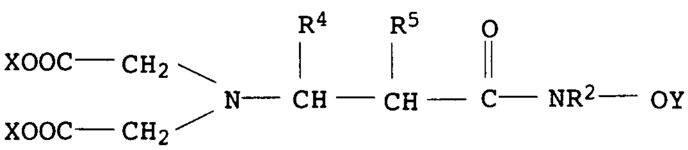

(IIIa)

where

$R^4$ is hydrogen, methyl or ethyl,

$R^5$ is hydrogen, methyl, ethyl or hydroxyl, and

$R^2$, X and Y have the abovementioned meanings.

**3.** A detergent or cleaner containing one or more hydroxamic acids or hydroxamic acid ethers as set forth in claim 1 or 2 in an amount of from 0.01 to 50 % of the total weight.

**4.** A detergent or cleaner as claimed in claim 3, additionally containing a bleaching system in the conventional amount.

**5.** A hydroxamic acid or hydroxamic acid ether of the formula III or IV

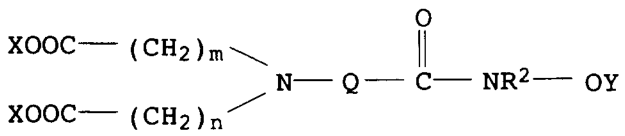

(III)

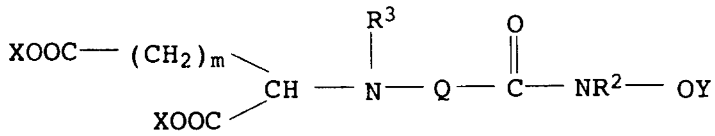

(IV)

where

$R^2$ is hydrogen or $C_1$-$C_{18}$-alkyl,

$R^3$ is hydrogen or a group of the formula —Q—CO—$NR^2$—OY,

Q is a $C_1$-$C_8$-alkylene group which can additionally carry up to 8 hydroxyl groups,

m and n are each 1 or 2,

X is hydrogen, alkali metal, ammonium or substituted ammonium, and

Y is hydrogen, alkali metal, ammonium, substituted ammonium or $C_1$-$C_{18}$-alkyl,

with the exception of N,N-bis(carboxymethyl)-2-aminoacetohydroxamic acid, N-carboxymethyl-N-(β-carboxyethyl)-2-aminoacetohydroxamic acid and N,N-bis(carboxymethyl)-3-aminopropiohydroxamic acid.

**6.** An N,N-bis(carboxymethyl)-3-aminopropiohydroxamic acid or its ether of the formula IIIa

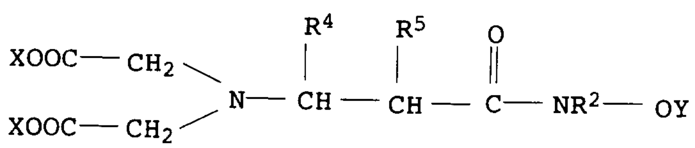

(IIIa)

where

$R^4$ is hydrogen, methyl or ethyl,

$R^5$ is hydrogen, methyl, ethyl or hydroxyl,

$R^2$ is hydrogen or $C_1$-$C_{18}$-alkyl,

X is hydrogen, alkali metal, ammonium or substituted ammonium, and

Y is hydrogen, alkali metal, ammonium, substituted ammonium or $C_1$-$C_{18}$-alkyl.

with the exception of N,N-bis(carboxymethyl)-3-aminopropiohydroxamic acid.

**7.** A process for preparing N,N-bis(carboxymethyl)-3-aminopropiohydroxamic acids and their ethers IIIa as set forth in claim 2, which comprises reacting iminodiacetic acid of the formula VI

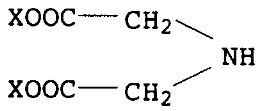

(VI)

with an α,β-unsaturated carboxylic ester of the general formula VII

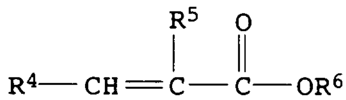

(VII)

or with an α,β-unsaturated carboxamide of the general formula VIII

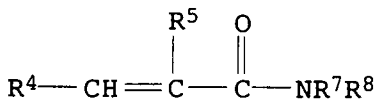

(VIII)

to give the N,N-bis(carboxymethyl)-3-aminopropionic ester of the general formula IX

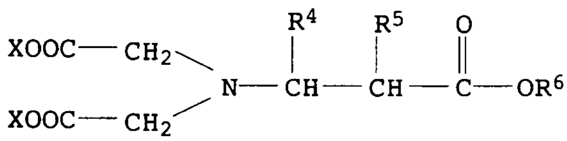

(IX)

or the N,N-bis(carboxymethyl)-3-aminopropionamide of the general formula X

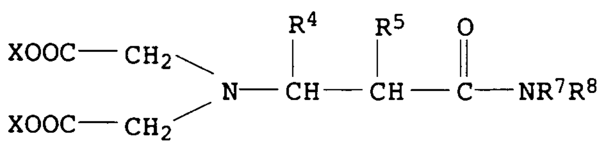

(X)

and subsequently reacting (IX) or (X) with a hydroxylamine or a hydroxylamine $C_1$-$C_{18}$-alkyl ether to give the final product IIIa which can, if required, be converted into the salt form by treatment with the bases from which X and Y are derived, with the variables $R^4$, $R^5$, X and Y having the abovementioned meanings, and $R^6$ being $C_1$-$C_4$-alkyl, and $R^7$ and $R^8$ being hydrogen or $C_1$-$C_4$-alkyl.

**8.** A mixture of

A) hydroxamic acids or hydroxamic acid ethers as set forth in claim 1 and

B) polycarboxylates in the form of

(i) homo-, co- or terpolymers of acrylic or methacrylic acid,
(ii) homo-, co- or terpolymers of aspartic acid,
(iii) homo-, co- or terpolymers of vinyl citrate or
(iv) low molecular weight mono- to hexacarboxylic acids

as free acids or as alkali metal, ammonium or substituted ammonium salts,

in the A:B ratio by weight of from 50:1 to 1:20.

**9.** A mixture of

A) N,N-bis(carboxymethyl)-3-aminopropiohydroxamic acids or their ethers of the general formula IIIa as set forth in claim 2 and

B) alkali metal salts of citric acid

in the A:B ratio by weight of from 20:1 to 1:20.

## Revendications

**1.** Utilisation d'acides hydroxamiques et d'éthers d'acides hydroxamiques de formules générales III et IV

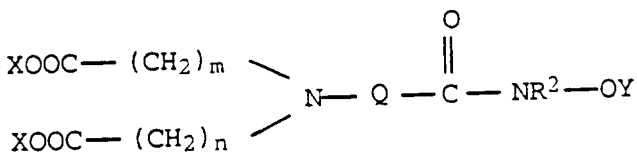

(III)

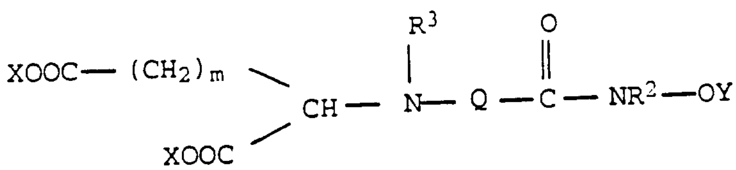

(IV)

dans lesquelles

| | |
|---|---|
| $R^2$ | représente un atome d'hydrogène ou un groupement alkyle en $C_1$-$C_{18}$, |
| $R^3$ | représente un atome d'hydrogène ou un groupement de formule -Q-CO-$NR^2$-OY, |
| Q | est mis pour un groupement alkylène en $C_1$-$C_8$ pouvant porter en outre jusqu'à 8 groupements hydroxy, |
| m et n | sont mis chacun pour le nombre 1 ou 2, |
| X | représente un atome d'hydrogène, de métal alcalin, un groupement ammonium ou ammonium substitué et |
| Y | représente un atome d'hydrogène, de métal alcalin, un groupement ammonium, ammonium substitué ou alkyle en $C_1$-$C_{18}$, |

en tant qu'adjuvants et qu'agents stabilisant de blanchiment pour des agents de lavage et de nettoyage.

**2.** Utilisation selon la revendication 1, d'acides N,N-bis(carboxyméthyl)-3-aminopropiohydroxamiques et de leurs éthers de formule générale IIIa

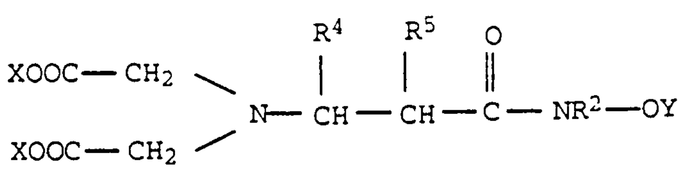

(IIIa)

dans laquelle

| | |
|---|---|
| $R^4$ | est mis pour un atome d'hydrogène, un groupement méthyle ou éthyle, |
| $R^5$ | est mis pour un atome d'hydrogène, un groupement méthyle, éthyle ou hydroxy et |
| $R^2$, X et Y | prennent la signification susmentionnée. |

3. Agents de lavage et de nettoyage contenant un ou plusieurs acides hydroxamiques ou éthers d'acides hydroxamiques selon la revendication 1 ou 2, en une quantité de 0,01-50% en poids, par rapport au poids total.

4. Agents de lavage et de nettoyage selon la revendication 3, contenant en outre un système d'agents de blanchiment en quantité usuelle.

5. Acides hydroxamiques et éthers d'acides hydroxamiques de formules générales III et IV

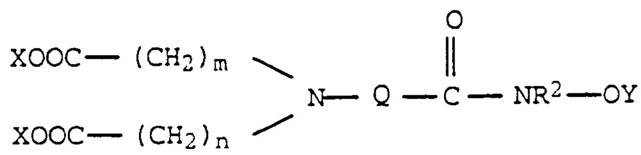

(III)

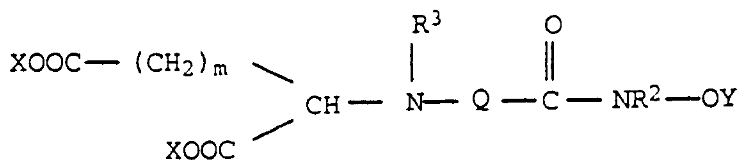

(IV)

dans lesquelles

| | |
|---|---|
| $R^2$ | représente un atome d'hydrogène ou un groupement alkyle en $C_1$-$C_{18}$, |
| $R^3$ | représente un atome d'hydrogène ou un groupement de formule -Q-CO-$NR^2$-OY, |
| Q | est mis pour un groupement alkylène en $C_1$-$C_8$ pouvant porter en outre jusqu'à 8 groupements hydroxy, |
| m et n | sont mis chacun pour le nombre 1 ou 2, |
| X | représente un atome d'hydrogène, de métal alcalin, un groupement ammonium ou ammonium substitué et |
| Y | représente un atome d'hydrogène, de métal alcalin, un groupement ammonium, ammonium substitué ou alkyle en $C_1$-$C_{18}$ |

à l'exception de l'acide N,N-bis(carboxyméthyl)-2-aminoacéthydroxamique, de l'acide N-carboxyméthyl-N-(β-carboxyéthyl)-2-aminoacéthydroxamque et de l'acide N,N-bis(carboxyméthyl)-3-aminopropiohydroxamique.

6. Acides N,N-bis(carboxyméthyl)-3-aminopropiohydroxamiques et leurs éthers de formule générale IIIa

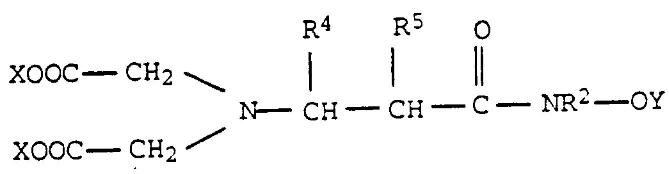

(IIIa)

dans laquelle

| | |
|---|---|
| $R^4$ | est mis pour un atome d'hydrogène, un groupement méthyle ou éthyle, |
| $R^5$ | est mis pour un atome d'hydrogène, un groupement méthyle, éthyle ou hydroxy |
| $R^2$ | est mis pour un atome d'hydrogène, ou un groupement alkyle en $C_1$-$C_{18}$, |
| X | représente un atome d'hydrogène, de métal alcalin, un groupement ammonium ou ammonium substitué et |
| Y | représente un atome d'hydrogène, de métal alcalin, un groupement ammonium, ammonium substitué ou alkyle en $C_1$-$C_{18}$, |

à l'exception de l'acide N,N-bis(carboxyméthyl)-3-aminopropiohydroxamique.

**7.** Procédé de préparation d'acides N,N-bis(carboxyméthyl)-3-aminopropiohydroxamiques et de leurs éthers IIIa selon la revendication 2, caractérisé en ce que l'on fait réagir un acide iminodiacétique de formule VI

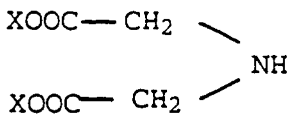

(VI)

avec un ester d'acide carboxylique α,β-insaturé de formule générale VII

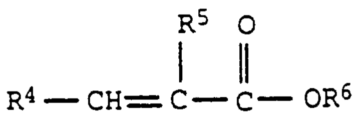

(VII)

ou un amide d'acide carboxylique α,β-insaturé de formule générale VIII

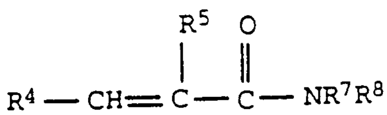

(VIII)

ce qui donne un ester d'acide N,N-bis(carboxyméthyl)-3-aminopropionique de formule générale IX

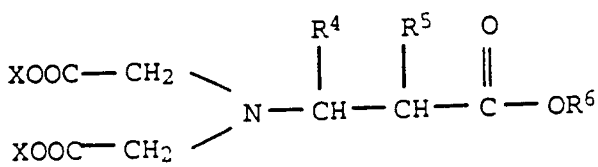

(IX)

ou un amide d'acide N,N-bis(carboxyméthyl)-3-aminopropionique de formule générale X

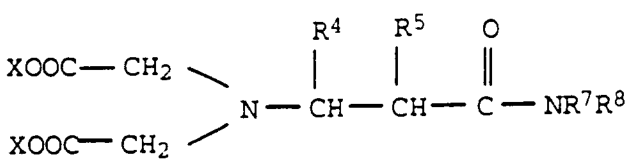

(X)

et on prépare le produit final IIIa à partir de (IX) ou (X) par réaction avec une hydroxylamine ou avec une hydroxylamine-(alkyle en $C_1$-$C_{18}$)éther, ce produit final pouvant être mis sous forme de sel par traitement avec les bases correspondant à X ou Y, les variables $R^4$, $R^5$, X et Y ayant les significations susmentionnées, $R^6$ représentant un groupement alkyle en $C_1$-$C_4$, et $R^7$ et $R^8$ représentant un atome d'hydrogène ou un groupement alkyle en $C_1$-$C_4$.

**8.** Mélanges constitués

A) d'acides hydroxamiques ou d'éthers d'acides hydroxamiques selon la revendication 1 et
B) de polycarboxylates sous forme de

(i) homo-,co- ou terpolymères d'acide acrylique ou méthacrylique,
(ii) homo-,co- ou terpolymères d'acide aspartique,

(iii) homo-,co- ou terpolymères de citrate de vinyle ou
(iv) d'acides mono- à hexacarboxyliques à bas poids moléculaire,

en tant qu'acides libres ou en tant que sels de métal alcalin, d'ammonium ou d'ammonium substitué,

dans un rapport en poids A : B de 50 : 1 à 1 : 20.

**9.** Mélanges constitués

A) d'acides N,N-bis(carboxyméthyl)-3-aminopropiohydroxamiques ou de leurs éthers de formule générale IIIa selon la revendication 2 et
B) de sels de métal alcalin d'acide citrique

dans un rapport en poids A : B de 20 : 1 à 1 : 20.